# EUROPEAN PATENT SPECIFICATION

(11) **EP 1 764 616 B1**
(45) Date of publication and mention of the grant of the patent: **08.04.2009**
(21) Application number: 06026484.3
(22) Date of filing: 15.06.2001
(51) Int. Cl.: G01N 33/68, G01N 33/50

(54) **Method for identifying apoptosis modified proteins**
Verfahren zur Identifizierung während der Apoptose modifizierter Proteine
Procédé pour l'identification de protéines modifiées durant l'apoptose

(30) Priority: 16.06.2000 EP 00112813; 16.11.2000 EP 00125013
(43) Date of publication of application: 21.03.2007
(62) Divisional of application: 01943510.6
(73) Proprietor: Rudel, Thomas, Dr., 13591 Berlin (DE)
(72) Inventor: Rudel, Thomas, Dr., 13591 Berlin (DE); Thiede, Bernd, Dr., 0258 Oslo (NO); Machuy, Nikolaus, 10439 Berlin (DE)
(74) Representative: Weiss, Wolfgang

(56) References cited:
- WELBURN SUSAN C ET AL: "Prohibitin and RACK homologues are up-regulated in trypanosomes induced to undergo apoptosis and in naturally occurring terminally differentiated forms" CELL DEATH AND DIFFERENTIATION, vol. 5, no. 7, July 1998 (1998-07), pages 615-622, XP008074838 ISSN: 1350-9047
- THOMPSON WINSTON E ET AL: "Immunolocalization and expression of prohibitin, a mitochondrial associated protein within the rat ovaries" ANATOMICAL RECORD, vol. 256, no. 1, 1 September 1999 (1999-09-01), pages 40-48, XP008074843 ISSN: 0003-276X
- MCCLUNG J K ET AL: "PROHIBITIN: POTENTIAL ROLE IN SENESCENCE, DEVELOPMENT, AND TUMOR SUPPRESSION" EXPERIMENTAL GERONTOLOGY, ELSEVIER SCIENCE, OXFORD, GB, vol. 30, no. 2, March 1995 (1995-03), pages 99-124, XP002073862 ISSN: 0531-5565
- DAHM RALF ET AL: "Changes in the nucleolar and coiled body compartments precede lamina and chromatin reorganization during fibre cell denucleation in the bovine lens" EUROPEAN JOURNAL OF CELL BIOLOGY, vol. 75, no. 3, March 1998 (1998-03), pages 237-246, XP008074844 ISSN: 0171-9335
- DATABASE EMBL 1992, "prohibitin [Homo sapiens]" XP002419085 Database accession no. AAB21614
- FUSARO GINA ET AL: "Prohibitin induces the transcriptional activity of p53 and is exported from the nucleus upon apoptotic signaling." JOURNAL OF BIOLOGICAL CHEMISTRY, vol. 278, no. 48, 28 November 2003 (2003-11-28), pages 47853-47861, XP008074839 ISSN: 0021-9258

## Description

The present invention relates to a screening method for identifying an apoptosis modulator, characterized in that the method comprises determination of transcriptional, translational, posttranslational variation and/or translocation from one compartment to another of prohibitin in a cell during apoptosis, wherein the apoptosis modulator is capable of modifying prohibitin during apoptosis.

Apoptosis is an essential and complex process for the development and homeostasis of multicellular organisms. Improper regulation of this process results in various diseases including cancer, autoimmune disorders, viral infections, neurodegenerative disorders and myocardial infarction (1). The therapeutic regulation of apoptosis therefore offers numerous challenges (2).

Several component of the apoptotic cell death machinery were already identified. The best known contributors are the caspases (3,4) and their inhibitors (5) and substrates (6), the bcl-2 family (7,8), the death receptors (9), the mitochondria (10,11) and signal transduction pathways (12,13). Death receptors belong to the tumour necrosis factor (TNF) superfamily. The best characterized death receptors are Fas, TNFR1, DR3, DR4 and DR5. These receptors induce apoptosis by ligand binding and receptor oligomerization, recruitment of an adaptor protein to the death domain of the receptor. The adaptor molecule binds a caspase, thereby activating the apoptosis machinery. On the other hand decoy receptors compete with specific death receptors for ligand binding.

However, hundreds of stimuli induce apoptosis independent of death-receptor like UV or γ-irradiation, chemotherapeutic drugs and viral or bacterial infections. The apoptotic phenotype is very similar in all apoptotic cells independent of the stimuli used to induce apoptosis. In addition, apoptosis of cells from organisms which are evolutionary distantly related, like nematodes and man, is regulated by structurally related proteins like caspases and these cells show similar phenotypes. These findings together were the basis for a concept of a highly conserved apoptotic machinery involving similar factors in all cells.

The Fas receptor (CD95 or Apo1) plays an important role in immune regulation by deletion of autoimmune cells and activation-induced -T-cell death, killing of targets such as virus-infected cells or cancer cells by cytotoxic T-cells and by natural killer cells and killing of inflammatory cells at immune privileged sites (14-16). Fas is expressed in a wide variety of cells, whereas the Fas ligand (FasL) has a limited tissue distribution. FasL is rapidly induced in activated T-cells and natural killer cells but few other cells appear to express significant levels of FasL. The decoy receptor DcR3 binds to FasL and inhibits FasL-induced apoptosis (17). Thus, tumours may be able to evade the death signal by binding of a trigger of apoptosis. Cisplatin causes intra-DNA strand cross links. DNA damage induced by cisplatin ultimately induces apoptosis in a variety of cell lines.

Welburn et al. (Cell. Death and Differentiation 1998, 5:615-622) refers to trypanosomes treated with concanavalin A resulting in an upregulation of prohibitin. There is no indication in this document that concanavalin A induced apoptosis results in a modification of prohibitin. -

Thompson et al. (The Anatomical Record 1999, 256:40-48) refers to apoptosis induction by gonadotropin releasing hormone agonist GnRH-Ag, resulting in an increase in prohibitin expression. There is no indication in this document that prohibitin may by modified during apoptosis.

Proteome approaches have been used to find new apoptosis-associated proteins (18). However, the conditions used in these studies to induce apoptosis allowed synthesis of new proteins because (1) protein synthesis was not blocked by the addition of protein synthesis inhibitors such as cycloheximide and (2) the cells were stimulated to undergo apoptosis for such a long time (more than 12 h) that synthesis of new proteins was possible. The modified proteins obtained by this treatment thus consisted of apoptosis-modified proteins and proteins which were expressed as a. general response of the cell to stress. The identification of a protein as apoptosis-modified was thus not possible.

The object underlying the present invention was-to provide targets for the identification of apoptosis modulators, and a method for identification of apoptosis modulators related to these targets.

In order to solve this problem we induced apoptosis by the addition of Anti-Fas IgM antibody in a defined way for 6 h or cis-platin for 16 h and at the same time blocked the synthesis of new proteins by the addition of cycloheximide. Under these conditions only apoptosis-modified proteins, and not newly synthesised proteins, were detected. This is also very important for the apoptosis-induced translocation of proteins which can be attributed to the movement of a pre-formed protein upon apoptosis induction. Translocation from the cytosol to the nucleus in apoptotic cells of the pre-formed caspase activated DNAse (CAD) is shown in (33).

Translocation of Bid from the cytosol to the mitochondria is the critical event in Fas-induced apoptosis in several cell lines. Thus interference with apoptosis-induced translocation of proteins might be of therapeutic use to either trigger apoptosis in proliferative diseases or to, prevent apoptosis in degenerative diseases.

Thus, the present invention provides a proteome analysis of cells to characterize and/or identify apoptosis-associated and particularly apoptosis-modified proteins. Subtractive analysis of two dimensional gel-electrophoresis patterns of apoptotic cells and non-apoptotic cells revealed differences in a plurality of protein spots. The predominantly altered protein spots were identified after proteolytic digestion and peptide mass fingerprinting. Of the identified proteins, the heterogeneous nucleoprotein (hnRNP) A/B, hnRNP A2/B1, hnRNP A3, hnRNP D, hnRNP F, hnRNP H, hnRNP I, hnRNP K, hnRNP L, hnRNP R, hnRNP JKTBP1, hnRNP A0, and Apobec-1 interacting protein, the splicing factors SRp30c, P54nrb, SF2p33 (ASF-2), SF SC35, NMP200 (related to SR PRP19) and PTB-associated SF, splicing factor 1, and KH-type splicing regulatory protein, the translation factors EF-Tu, EF-1 beta, EIF-5A, 40 S ribosomal protein SA, elongation factor 1-delta, elongation initiation factor 3 (subunit 4) and poly(A)-binding protein (cytoplasmic 4), the structural proteins gamma-actin and the myosin heavy chain, the factors involved in signal transduction GAP SH3 binding protein, cGMP-dependent protein kinase, GAP SH3 protein 2, and the small G protein, the chromatins type I alpha, Baf-57, CAF-1 (RB b.p.) (WD-repeats) and KIAA1470; the transcription factor CBF-beta, the proteasomal factor 26S protease SU 12, proteasome subunit C8 and Tat binding protein-1, the mitochondrial factors isocitrate dehydrogenase, AOP-1, ATP synthase beta chain and ATP synthase D chain and the diverse factors SYT interacting protein SIP, PA1-G, CRHSP-24, HCD2, GMP synthase, FUSE binding protein 1, HDGF, alpha NAC, ARDH, cargo selection protein, DAZ associated protein 1, DEAD box protein retinoblastoma, dihydrofolate reductase, hydroxyacyl-CoA dehydrogenase/3-ketoacyl-CoA thiolase/enoyl-CoA hydratase, ER-60, HCA56, Hsp-105, IGF-II mRNA-binding protein 1, IGF-II mRNA-binding protein 3, lactate dehydrogenase A, NS-associated protein, RAD 21, RAD 23 homolog B, T-complex protein 1 beta subunit, thioredoxin like protein, an unnamed protein (NCBI 7020309), chondrosarcoma-associated protein 2, ELAV-like 1 (Hu antigen R), HnRNP M, HnRNP E1, SKI interacting protein, glutathione S-transferase, VDAC 3, mortalin-2 (heat shock 70 kd protein 9B), prohibitin, 26S protease regulatory subunit 4, and proteasome subunit alpha type 1 were hitherto unknown to be involved in apoptosis. HnRNP C1/C2, nucleolin, p54nrb, Rho GD12, ASF-2, SRp30c and BTF3 include aspartic acid/glutamic acid-rich domains and hn RNP A2/B1, hn RNP C1/C2, nucleolin and BTF3 interact with protein kinase CK2. Remarkably, the heterogeneous nuclear ribonucleoprotein (hnRNP) A/B, hnRNP A1, hnRNP A2/B1, hnRNP A3, hnRNP C1/C2, hnRNP D, hnRNP F, hnRNP H, hnRNP I, hnRNP K, hnRNP L, hnRNP R, hnRNP JKTB1, the splicing factors SRp30c, P54nrb, SF2p33 (ASF-2), SFSC35, PTB-associated SF, the signal transduction protein GAP SH3 binding protein, the chromatin associated protein nucleolin, hnRNP A0, Apobec-1 interacting protein, elongation initiation factor 3 (subunit4), poly(A)-binding protein (cytoplasmic4), GAP SH3-binding protein 2, DAZ associated protein 1, IGF-II mRNA-binding protein 1, IGF-II mRNA-binding protein 3, NS-associated protein, Hn RNP M and ELAV-like 1 contain the RNP motif. The proteins splicing factor 1, KH-type splicing regulatory protein, IGF-II mRNA-binding protein 1, IGF-II mRNA-binding protein 3 and Hn RNP E1 contain the KH motif. Prohibitin is known to be an inhibitor of DNA synthesis, Hsp-60 and Mortalin-2 are known to be chaperones. VDAC 3 is known to be an ion channel. The proteins PFC6D, KNFE3 (partial sequence TPGT(F/Mox)E) and KPF1 were unknown.

The apoptosis-modified protein employed in the method of the present invention is prohibitin.

'Modification' or 'apoptosis-modified' in this context describes the alteration of a protein in a given compartment during the process of apoptosis. The protein spot elicits changes in the size or the charge or the size and the charge. These changes may be due to transcriptional (e.g. splicing), translational and/or posttranslational (e.g. glycosylation and/or proteolyis) variations. Furthermore, the protein may be translocated. 'Translocation' in this context describes differences in the localisation of a protein in compartments of apoptotic cells compared to the compartments of non-apoptotic cells.

The method established and described above can be used for other cell types expressing death receptors like TNF-receptor, DR-3, DR-4 or DR-5 or any receptor which induces apoptosis in the absence of protein biosynthesis. The method can be used for cells induced to undergo apoptosis by other pathways than the receptors described above.

Thus, a disclosed matter is a method for characterizing and/or identifying apoptosis-modified proteins comprising the steps:
(a) providing a first extract and a second extract comprising soluble proteins, wherein said first extract is from a cell without apoptosis induction and said second extract is from a cell after apoptosis induction,
(b) separating said first and second extracts by two-dimensional gel electrophoresis, wherein said first and second proteome patterns each comprising a plurality of protein species are obtained
(c) comparing said first and second proteome patterns and
(d) characterizing and/or identifiying apoptosis-modified protein species.

In the context of the present application, characterization of a protein is the analysis of the chemical composition of the protein. Identification of a protein is the assignment of a spot on the 2-DE gel to its biological functions or at least the assignment to a gene including the regulatory encoding sequences. In the context of the present invention, the proteome comprises the protein composition of a cell or a part of it at a defined biological situation (19).

The method for characterizing und/or identifying apoptosis-modified proteins allows characterization and identification of apoptosis-modified proteins from cells, preferably from mammalian cells, more preferably from human cells, such as mammalian and particularly human T-cells, e.g. from an immortalized T-cell line such as the T-cell line Jurkat E6 (ATCC TIB 152).

Step (a) of the method for characterising and/or identifying apoptosis-modified proteins comprises the preparation of extracts comprising soluble proteins. A first extract is obtained from a cell without apoptosis induction and a second extract is obtained from a cell after apoptosis induction. The extracts may be whole cell extracts but may be also extracts from cell compartments such as membranes, cytosol, mitochondrial or nucleus. Apoptosis may be induced by contacting the cells with caspase activators and/or ligands of death receptors (such as an anti-Fas antibody) and/or cis-platin.

Preferably, the second extract is obtained from a cell wherein after apoptosis induction substantially no synthesis of new proteins has been allowed. This may be effected by adding an inhibitor of protein biosynthesis such as cycloheximide and/or by carrying out apoptosis induction for a period of time which is too short to allow a substantial synthesis of new proteins, e.g. a period of time of less than 12 h, preferably less than 8 h, e.g. about 6 h.

Step (b) of the method for characterising and/or identifying apoptosis modified proteins is a two-dimensional gel electrophoresis which comprises (i) separation in a first dimension according to the isoelectric point and (ii) separation in a second dimension according to size. The gel matrix is preferably a polyacrylamide gel. Gel preparation may be carried out according to known methods (20,21).

Step (c) of the method for characterizing and/or identified apoptosis-modified proteins comprises comparing said first and second proteome patterns. This comparison may comprise a subtractive analysis of the first and second proteome patterns (22). By means of this subtractive analysis apoptosis-modified protein species are obtained which may be selected from protein species which (i) are located at different positions on the two-dimensional gels from the first and second extracts and/or (ii) have a different intensity on the two-dimensional gels from the first and second extracts.

The characterization of apoptosis-modified protein species may be carried out by peptide fingerprinting, wherein peptide fragments of the protein to be analysed are generated by in-gel proteolytic digestion, e.g. by digestion with trypsin. Further characterization of the peptides may be carried out by mass spectrometry, e.g. electrospray ionization mass spectrometry (ESI-MS) (23) and matrix-assisted laser dissorption/ionization mass spectrometry (MALDI-MS) (24) and/or by at least partial amino acid sequencing, e.g. by Edman degradation.

The method for characterizing and/or identifying apoptosis-modified proteins may further comprise as step (e) the
determination if the apoptosis-associated modifications of the protein species are present in subjects, e.g. experimental animals or human patients suffering from apoptosis-associated diseases including hyperproliferative or degenerative diseases such as cancer, autoimmune and neurodegenerative disorders such as Alzheimer's diseases, viral infections such as AIDS and vascular diseases such as myocardial infarction. By screening the presence of apoptosis-modified proteins in the patients, valuable targets for preventing or treating the above diseases may be identified.

A still further subject matter of the present disclosure are individual proteins which are expressed by apoptotic cells, e.g. by apoptotic T-cells, and which have been characterized and identified by the method as described above. Preferably, these proteins are selected from heterogenous nuclear ribonucleoproteins such as hnRNP A/B (Gene bank Accession Number NM_004499), A1 (X12671), A2/B1 (D28877), A3 (AF148457), C1/C2 (NM_004500), D (D55671), F (L28010), H (L22009), I (NM_002819), K (NM_002140), L (NM_001533), R (AF000364), JKTBP1 (D89092), hnRNP A0 (NM_006805) and Apobec-1 interacting protein (U76713), splicing factors such as SRp30c (NM_003769), p54nrb (U89867), SF2p33 (ASF-2) (M72709), SFSC35 (X62447), NMP200 (AJ131186), PTB-associated SF (NM-05066), splicing factor 1 (Y08766), and KH-type splicing regulatory protein (NM_003685), translational factors such as 60S acidic ribosomal protein (NM_001002), EF-Tu (N_003321), EF-1β (NM_001959), EIF-5A (NM_001970), 40 S ribosomal protein SA (NM_002295), elongation factor 1-delta (NM_001960), elongation initiation factor 3 (subunit 4, AF020833), and poly(A-)binding protein (cytoplasmic 4, NM_003819), structural proteins such as lamin B1 (L37747), lamin B2 (M94362), vimentin (NM_003380) and beta-tubulin (VO0599), the structural proteins gamma actin (M19283) and the myosin heavy chain (M31013), signal transduction proteins such as GAP SH3 binding protein (NM_005754), Rho GD12 (X69549), cGMP-dependent protein kinase type Iα (Z92867), GAP SH3 protein 2 (AF051311), and the small G protein (NM_002872), chromatin associated proteins such as nucleolin (NM_005381), Baf-57 (NM_003079), CAF-1 (X71810), and KIAA 1470 (AB040903), transcription factors such as BTF3 (X53281) and CBF-β (L20298), proteasome subunits such as 26S protease subunit12 (NM_002811), proteasome subunit C8 (NM_002788) and Tat binding protein-1. (NM_02804), mitochondrial proteins such as isocitrate dehydrogenase (NM_002168), AOP-1 (NM_006793), ATP synthase beta chain (M27132), ATP synthase D chain (NM_006356), nucleophosmin (X16934), SYT interacting protein SIP (NM_006328), PA1-G (NM_002573). CRHSP-24 (AF115345), HCD2 (NM_004493), GMP synthase (NM_003875), FUSE binding protein 1 (NM_003902), HDGF (NM_004494), alpha NAC (NM_005594), ARDH (X77588), cargo selection protein (NM_005817), DAZ associated protein 1 (NM_018959), DEAD box protein retinoblastoma (NM_004939), dihydrofolate reductase (NM_000791), hydroxylacyl-CoA dehydrogenase/3-ketoacyl-CoA thiolase/enoyl-CoA hydratase (NM_000182), ER-60 (NM_005313), HCA56 (AF220417), Hsp-105 (NM_006644), IGF-II mRNA-binding protein 1 (NM_006546), IGF-II mRNA-binding protein 3 (AF117108), lactate deydrogenase A (NM_005566), NS-associated protein (NM_006372), RAD 21 (X98294), RAD 23 homolog B (NM_002874), T-complex protein 1 beta subunit (U91327), thioredoxin like protein (NM_004786), an unnamed protein (NCBI 7020309. AK000310), c-Abl (P00519, pl 8.8, MW .140 kDa, determined by 2 DE gel electrophoresis), alpha-fodrin, Hsp-60, chohdrosarcoma-associated protein 2, ELAV-like 1 (Hu antigen R), HnRNP M, HnRNP E1, SKI interacting protein, glutathione S-transferase, VDAC 3, mortalin-2 (heat shock 70 kd protein 9B), prohibitin, 26S protease regulatory subunit 4, and proteasome subunit alpha type 1. More preferably, these proteins are selected from the proteins as shown in Table 1 and the new proteins PFC6D, KNFE3 (partial sequence TPGT(F/Mox)E) and KPF1.

A subject matter of the present disclosure are proteins translocated from one cellular compartement such as nucleus, cytosol, mitochondria or membrane to another. Preferably, these proteins are selected from the protein species as described in Tables 3, 4, 5, 6, 7 or 8.

Especially preferred is the apoptosis-associated and/or -modified protein prohibitin.

Subject of the present invention is the use of prohibitin in a method for identifying apoptosis modulators, wherein the apoptosis modulator is capable of modifying prohibitin during apoptosis.

A further subject of the present invention is a screening method for identifying an apoptosis modulator, characterized in that the method comprises determination of transcriptional, translational, posttranslational variation and/or translocation from one compartment to another of prohibitin in a cell during apoptosis, wherein the apoptosis modulator is capable of modifying prohibitin during apoptosis.

In addition to the proteins as specified above or fragments thereof having a length of preferably at least 10, more preferably at least 20 and most preferably at least 30 amino acids, the disclosure also relates to nucleic acids, e.g. DNA, RNA or nucleic acid analogs, e.g. DNA which encode these proteins or protein fragments or variants, e.g. allelic variants thereof.

The proteins or protein patterns as described above may be used as targets for the diagnosis, prevention or treatment of apoptosis-associated diseases or in a method for identifying apoptosis-modulators. A diagnostic method may comprise a determination of the presence or absence of apoptosis-modified proteins in a sample. A preventive or therapeutic method may comprise the activation or inhibition of apoptosis-modified proteins, e.g. an activation by overexpression via gene transfer into cells or organs by gene transfer vectors such as viruses, an inhibition by antisense or ribozyme molecules or an activation or inhibition by substances which modulate the amount, processing, presentation or conformation of the protein. The method for identifying apoptosis modulators (activators or inhibitors) may comprise a screening assay, e.g. a cellular or molecular screening assay which may be carried out in a high-throughput format.

Apoptosis modulators which are identified by the method of the present invention or compounds derived therefrom, e.g. by empirical derivatization and/or by computer modelling, may be provided as pharmaceutical compositions optionally together with suitable pharmaceutically acceptable carriers, diluents and/or adjuvants.

The proteins described in this application or proteins identified by the method described above can be used to develop modification-specific diagnostic tools such as antibodies or phages or other substances. The proteins or useful fragments can be used to develop protein chips or other solid-phase screening devices for high throughput screens.

The proteins identified by this technique are potential targets for diseases associated with apoptosis. Such diseases are tumours which can be associated with identified proteins as GAP SH3 binding protein (NM_005754), Baf-57 (4507089), CAF-1 (422892), CBF-beta (2498753), AOP-1 (5802974), SYT interacting protein SIP (5454064), PA1-G (4505587), CRHSP-24 (4583307), FUSE binding protein 1 (4503801), HDGF (4758516), HCA56 (7678701), alpha NAC (NM 005594), ARDH (X77588), DEAD box protein retinoblastoma (NM 004939), HSP-105 (NM_006644), IGF-II mRNA binding protein 1 (NM_ 006546), IGF-II mRNA binding protein 3 (AF117108), RAD 21 (X98294), RAD 23 homolog B (NM_002874), thioredoxin like protein (NM_004786), hnRNP A/B (4758542), HnRNP A0 (8134660), hnRNP A1 (296650), hnRNP A2/B1 (565643), hnRNP A3 (6164674), hnRNP C1/C2 (4758544), hnRNP D (870743), hnRNP E1 (2134737), hnRNP F (452048), hnRNP H (347314), hnRNP 1 (4506243), hnRNP K (4504453), hnRNP L (4557645), hnRNP M (5174611), hnRNP R (2697103), Apobec-1 interacting protein (1814274), JKTBP1 (2780748), SRp30c (4506903), p54nrB (1895081), SF2p33 (ASF-2, 179074), SF SC35 (35597), NMP200 (5689738), splicing factor PTB (4826998), splicing factor 1 (16204031), KH-type splicing regulatory protein (FUSE binding protein 2, 2460200), DAZ associated protein (9506537), elongation initiation factor 3 subunit 4 (2460200), NS-associated protein 1 (5453806), nucleolin (4885511), poly(A)-binding protein cytoplasmic 4 (4504715), Ras-GAP SH3 binding protein (3098601), an unnamed protein product (7023323), chondrosarcoma associated protein 2 (5901878), ELAV-like protein 1 (4503551), SKI-interacting protein 1 (2500813), prohibitin (464371), nucleophosmin (114762), T-complex protein 1 beta subunit (1871210), heterochromatin protein p25 (5803076), KIAA1470 (7959201) and cAbl (125135). Further diseases are viral infections like HIV infection which can be associated with identified proteins as Tat binding protein-1 (4506211), CBF-beta (2498753) and EIF-5A (4503477). Further diseases are neurodegenerative diseases like Alzheimer's disease and Parkinson's disease which can be associated with identified proteins as HCD2 (4758504), AOP-1 (5802974), thioredoxin-related protein of 32 kDa (4759274), ERp37 (4885359), cGMP dependent protein kinase (6225588), VDAC-3 (5032221), HSP105 (5729879) and CRHSP-24 (4583307). Further diseases are ischemic stroke, heart failure and arthritis, which can be associated with identified protein AOP-1 (5802974), VDAC-3 (5032221), HSP105 (5729879), CRHSP-24 (4583307) and PAF acetylhydrolase (4505587).

Therefore the lack of expression or over-expression can be indicative of a disease and thus has diagnostic implications. The genes of the identified proteins can be used to develop DNA-chips or other DNA-or RNA-based screening devices (PCR, RT-PCR) to screen cells or tissues for the differences in the mRNA levels of the identified genes.

The present invention is to be further illustrated by the following figures and examples.

### Figure 1

2-DE gel of Fas-induced Jurkat T-cells (see Table 3).

### Figure 2

2-DE gel of Jurkat T-cells (control, see Table 3).

### Figure 3

2-DE gel of the cytosolic compartment of Fas-induced Jurkat T-cells (see Table 4).

### Figure 4

2-DE gel of the cytosolic compartment of Jurkat T-cells (control, see Table 4).

### Figure 5

2-DE gel of the nucleic compartment of Fas-induced Jurkat T-cells (see Table 5).

### Figure 6

2-DE gel of the nucleic compartment of Jurkat T-cells (control, see Table 5).

### Figure 7

2-DE gel of the mitochondrial compartment of Fas-induced Jurkat T-cells (see Table 6)

### Figure 8

2-DE gel of the mitochondrial compartment of Jurkat T-cells (control, see Table 6)

### Figure 9

Peptide mass fingerprinting of unknown protein called PFC6D (see Table 3). The peptide is characterized by fragments with the following masses: 1462.01, 1477.9, 1484.9, 1550.11, 1615.04, 2529.33, 2543.22 dalton.

### Figure 10

Peptide mass fingerprinting of unknown protein called KPF1 (see Table 5). The peptide is characterized by fragments with the following masses: 842.15, 992.529, 1006.57, 1092.58, 1109.6, 1274.68, 1288.68, 1265.76, 1249.58, 1338.73, 1455.74, 1564.74, 1758.93, 2004.03, 2034.09, 2080.96, 2110.75, 2211.09 and 2250.33 dalton.

### Figure 11

Peptide mass fingerprinting of unknown protein called KNFE3 (see Table 5). The peptide is characterized by fragments with the following masses: 696.42, 967.438, 1060.59, 1252.67, 1289.72, 1310.65, 1417.79, 1554.92, 1582.9, 1594.75, 1640.73, 1649.76, 1979.94, 1994.05 dalton.

### Figure 12

The partial sequence TPGT(F/Mox)E of the protein KNFE3 was obtained by ESI-MS/MS of the 1649,79 dalton fragment.

### Figure 13

2-DE gel of the membrane compartment of Fas-induced Jurkat T-cells (cf. Table 7)

### Figure 14

2-DE gel of the membrane compartment of Jurkat T-cells (control, cf. Table 7)

### Figure 15

2-DE gel of the total cell lysate of cis-platin induced apoptotic Jurkat T-cells (cf. Table 8).

### Figure 16

2-DE gel of the total cell lysate of Jurkat T-cells (control, cf. Table 8).

### Figure 17

2-DE gel of the mitochondrial compartment of cis-platin induced Jurkat T-cells (cf. Table 8). The control (mitochondrial compartment of non-induced T-cells) is not shown.

### Figure 18

2-DE gel of the membrane compartment of Jurkat T-cells (cf. Table 8). The membrane compartment of cis-platin induced Jurkat T-cells is not shown.

### Example

### 1. Materials and methods

### 1.1 Cell culture

The Jurkat T-cell line E6 (ATCC TIB 152) was maintained in RPMI tissue culture medium (Gibco BRL, Karlsruhe, Germany) supplemented with 10% fetal calf serum (Gibco BRL, Karlsruhe, Germany) and penicillin (100 U/ml)/streptomycin (100µg/ml) (Gibco BRL, Karlsruhe, Germany) at 37°C in 5.0% CO₂.

### 1.2 Induction of apoptosis

Apoptosis was induced to 2 x 10⁶ Jurkat T-cells for 6 h at 37°C in 5.0% CO₂ by 250 ng/ml aCD95 (clone CH 11) (Immunotech, Marseille, France) or for 16 h at 37°C in 5.0% CO₂ by 60µM cis-platinum(II)diaminedichloride (cis-platin, Sigma, Deisenhofen, Germany) in DMSO. 1 µg/ml cycloheximide was added to the control- and Fas induced cells, 0.5 µg/ml cycloheximide was added to the control- and cis-platin induced cells.

### 1.3 Separation of the compartments

Approximately 1×10⁸ Jurkat T cells were centrifuged for 10 min at 1300 U/min at room temperature in a Megafuge 1.0R (Heraeus, Hanau, Germany). The supernatant was discarded and the pellet was washed twice with 10 ml PBS (GibcoBRL, Karlsruhe, Germany) and once with MB buffer (400 mM sucrose, 50 mM Tris, 1 mM EGTA, 5 mM 2-mercaptoethanol, 10 mM potassium hydrogenphosphate pH 7.6 and 0.2% BSA) and centrifuged as above. The pellet was suspended in MB buffer (4 ml/10⁸ cells) and incubated on ice for 20 min. Subsequently the cells were homogenized and centrifuged at 3500 U/min for 1 min at 4°C (Rotor SS-34; Sorvall RC5B, Hanau, Germany). The supernatant contained the mitochondria/cytosol/membranes and the pellet enclosed the nucleus.

The mitochondrial fraction was pelleted by centrifugation at 8600 U/min for 10 min at 4°C (Rotor SS-34; Sorvall RC5B, Hanau, Germany). The supernatant contained the cytosol and membranes.

The pellet was suspended in MSM buffer (10 mM potassium hydrogenphosphate pH 7.2, 0.3 mM mannitol and 0.1% BSA) (0.4 ml/108 cells) and purified by sucrose gradient centrifugation in 10 ml SA buffer (1.6 M sucrose, 10 mM potassium hydrogenphosphate pH 7.5 and 0.1 % BSA) at 20000 U/min, 1 hour, 4°C (Rotor SW-28; Beckman L8-70M Ultracentrifuge, München, Germany). The interphase which contained the mitochondria was collected, suspended in 4 volumes of MSM buffer and centrifuged again at 15500 U/min for 10 min. at 4°C (Rotor SS-34; Sorvall RC5B, Hanau, Germany). The pellet was suspended in MSM buffer without BSA and could be stored at -70°C.

The supernatant with the cytosol and membrane was centrifuged at 100000 U/min, 20 min, 4°C (Rotor TLA120.2 rotor, Ultracentrifuge Optima TLX, Beckman, München, Germany). The pellet contained the membranes.

The pellet with the nucleus was suspended in 5 ml PBS and centrifuged for 2 min at 3500 U/min at 4°C (Rotor SS-34; Sorvall RC5B, Hanau, Germany). The pellet was suspended in NB buffer (10 mM Hepes pH 7.4, 10 mM KCI, 2 mM dithiothreitol (DTT) and 1 mM Pefabloc) (1 ml/10⁸ cells) and incubated for 1 hour on ice, subsequently homogenized and applied to 10 ml 30% sucrose in NB buffer. After the centrifugation with the Megafuge 1.0R (Heraeus, Hanau, Germany) at 2000 U/min for 10 min at 4°C, the pellet was washed twice with 6 ml NB buffer, centrifuged as above, suspended in 1 ml NB buffer, and centrifuged again at 10000 U/min for 10 minutes at 4°C (Rotor SS-34; Sorvall RC5B, Hanau, Germany). The pellet could be stored at -70°C.

### 1 .4 2-DE Gel Electrophoresis

The proteins were separated by a large gel 2-DE technique (gel size 30 cm x 23 cm) (28). The isoelectric focusing rod gels (diameter 1.5 mm or 2.5 mm) contained 3.5% acrylamide, 0.3% piperazine diacrylamide (Bio-Rad, Munich, Germany) and a total of 4% w/v carrier ampholytes WITAlytes pH 2-11 (WITA GmbH, Teltow, Germany). About 200 µg to 500 µg of protein were applied to the anodic side of the gel and focused at 8870 Vh. After focusing, the gels were equilibrated for 10 minutes in a buffer containing 125 mM Tris/phosphate, pH 6.8, 40% glycerol, 70 mM dithiothreitol (DTT), and 3% SDS. The equilibrated gels were frozen at -70°C. After thawing, the isoelectric focusing gels were immediately applied to SDS-PAGE gels, which contained 15% w/v acrylamide and 0.2% bisacrylamide. The SDS-PAGE system of Laemmli, 1970 was used, replacing the stacking gel by the equilibrated IEF gel. Electrophoresis was performed using a two-step increase of current, starting with 15 minutes at 120 mA, followed by a run of about 6 hours at 150 mA until the front reached the end of the gel.

### 1.5 Staining

### 1.5.1 Staining with Coomassie Blue R-250

Preparative gels were stained with Coomassie Brilliant Blue R-250 (Serva, Heidelberg, Germany). After fixation over night in 1 l 50% ethanol/10% acetic acid/40% water, the gel was stained for at least 5 hours in 1 l 50% methanol/10% acetic acid/40% water, 1 g Coomassie Blue R-250. The staining solution was removed and the gel was destained for 1 hour with 1 l 5% methanol/12.5% acetic acid/82.5% water. Subsequently, the gel was kept for 4 hours in aqueous 7% acetic acid and stored at 4°C in a plastic foil.

### 1.5.2 Staining with silver nitrate

Analytical gels were stained with silver nitrate. After fixation for at least one hour in 1 l 50% ethanol/10% acetic acid/40% water, the gel was incubated for 2 hours in 1 l 30% ethanol/0.5 M sodium acetate/0.5 glutaraldehyde/0.2% sodium thiosulfate. After washing with water twice for 20 minutes, the gel was stained with 1 l 0.1 % silver nitrate/0.01 % formaldehyde for 30 minutes. After washing for 30 seconds, the gel was developed for at least 4 minutes in 2.5% sodium carbonate, pH 11.3/0.05 mM sodium thiosulfate/0.01 % formaldehyde. The staining process was stopped by applying 0.05 M Titriplex III/0.02% Thimerosal. The solution was renewed after 15 minutes. Finally, the gels were dried for 3 hours at 70°C between cellophane membranes using a gel dryer (Model 585, Bio-Rad, München, Germany).

### 1.6 Tryptic digestion

The Coomassie Blue R-250 stained single gel spots from Jurkat T-cells were excised with a scalpel and shrunk by addition of 100 µl 50 mM ammonium bicarbonate, pH 7.8/acetonitrile (1:1) for 30 minutes at 37°C under shaking. Subsequently the solution was exchanged against 100 µl 50 mM ammonium bicarbonate, pH 7.8 for reswelling of the gel piece for 30 minutes at 37°C under shaking. The gel spots were dried in a vacuum concentrator (Eppendorf, Hamburg, Germany) after removing the buffer. 0.1 µg of trypsin (Promega, Madison, WI, USA) solved in 1 µl 50 mM acetic acid and 19 µl 50 mM ammonium bicarbonate, pH 7.8 were added. After incubation at 37°C for 16 hours the supernatant was removed and the gel pieces were washed with 20 µl 0.5% aqueous TFA/acetonitrile (2: 1) and again the supernatant was removed. The combined supernatants were evaporated in the vacuum concentrator and solved in 4 µl 0.5% aqueous TFA/acetonitrile (2: 1) for the mass spectrometrical analysis.

### 1.7 Peptide mass fingerprinting by MALDI-MS

The mass spectra were recorded by using a time-of-flight delayed extraction MALDI mass spectrometer (Voyager-Elite, Perseptive Biosystems, Framingham, MA, USA). The samples were mixed in an Eppendorf tube with the same volume of the matrix solution. Twenty mg/ml α-cyano-4-hydroxycinnamic acid (CHCA) in 0.3% aqueous TFA/acetonitrile (1:1) or 50 mg/ml 2,5-dihydroxybenzoic acid (DHB) in 0.3% aqueous TFA/acetonitrile (2: 1) were used as matrices. Two µl of the mixtures were applied to a gold-plated sample holder and introduced into the mass spectrometer after drying. The spectra were obtained in the reflectron mode by summing 100-200 laser shots with the acceleration voltage of 20 kV, 70% grid voltage, 0.05 guide wire voltage, 100 ns delay and the low mass gate at 500 m/z.

### 1.8 Sequencing by ESI-MS/MS

The mass spectra were aquired with a quadrupole/time-of flight ESI mass spectrometer equipped with a nebulized nanoelectrospray Z-spray source (Q-Tof, Micromass, Manchester, GB). Therefore, the tryptic digest was purified with a ZipTip C-18 tip (Millipore, Eschborn, Germany). The sample was evaporated and then dissolved in 2 µl 1 % acetic acid/49% water/50% methanol. Subsequently, 1 µl was introduced in the mass spectrometer using a nanospray needle to generate the mass spectra.

### 1.9 Database searching

The proteins were identified by using the peptide mass fingerprinting analysis software MS-Fit (http://prospector.ucsf.edu/ucsfhtml3.2/ msfit.htm). The NCBI database with the species human and mouse was used for the searches by considering at maximum one missed cleavage site, pyro-Glu formation at N-terminal Gln, oxidation of methionine, acetylation of the N-terminus and modification of cysteines by acrylamide.

The molecular masses and isoelectric points were calculated by employing the software Compute pl/Mw (http://www.expasy-ch/tool/pi_tool.html).

### 1.10 In vitro translation and cleavage assay

The cDNAs were translated in vitro using ³⁵S labelled methionine with the T-NT coupled reticulocyte lysate system according to the manufacturer's instructions (Promega, Mannheim, Germany). One µl of the translation product was cleaved with 3 µl active lysate or 20 U caspase-3 (BIOMOL, Hamburg, Germany) in 20 µl cleavage buffer (25 mM Hepes pH 7.5, 1 mM DTT, 1 mM EDTA and the protease inhibitors pefabloc pepstatin, leupeptin and aprotinin) for 1 h at 37°C. For inhibition experiments, 1 µl 5 mM Z-Val-Ala-DL-Asp-fluoromethylketone (Z-VAD-fmk) was added. The cleavage mixture was supplemented with 5 µl loading buffer (1 µl glycerol, 1 µl 10% SDS, 0.25 µl 2-mercaptoethanol, 0.075 mg Tris-base and 0.125 mg bromophenol blue) and applied to a 10% SDS-PAGE gel.

After electrophoresis, the gel was washed, dried and covered with a BioMax MR film (Kodak, Chalon-sur-Saone, France) overnight and then developed .

Active lysate was generated from Jurkat T-cells after 6 h induction of apoptosis with 250 ng/ml alphaCD95 (clone CH11, Immunotech, Marseille, France) and 1 µg/ml cycloheximide. Subsequently, the cells were washed with PBS and incubated for 20 min on ice with lysis buffer (25 mM Hepes, 0.1 % Chaps, 1 mM DTT and the protease inhibitors pefabloc, pepstatin, leupeptin and aprotinin).

Afterwards, the cells were homogenized and centrifuged for 5 min at 13.000 U/min (Biofuge fresco, Heraeus Instruments GmbH, Hanau, Germany). The supernatant was aliquoted and stored at -70°C.

In order to either verify or determine the cleavage by caspases, the cDNAs to be tested were cloned and expressed in vitro. The proteins were treated with either a lysate or apoptotic Jurkat T-cells which contained a mixture of active caspases, or with the recombinant purified caspase-3 in the presence or absence of the broad range caspase inhibitor zVAD-fmk. In most cases, the same cleavage pattern was observed for the proteins treated with the active lysate and caspase-3, however, the cleavage by caspase-3 was more efficient.

### 2. Results for the total cell lysate

### 2.1 Identification of apoptosis-modified protein spots

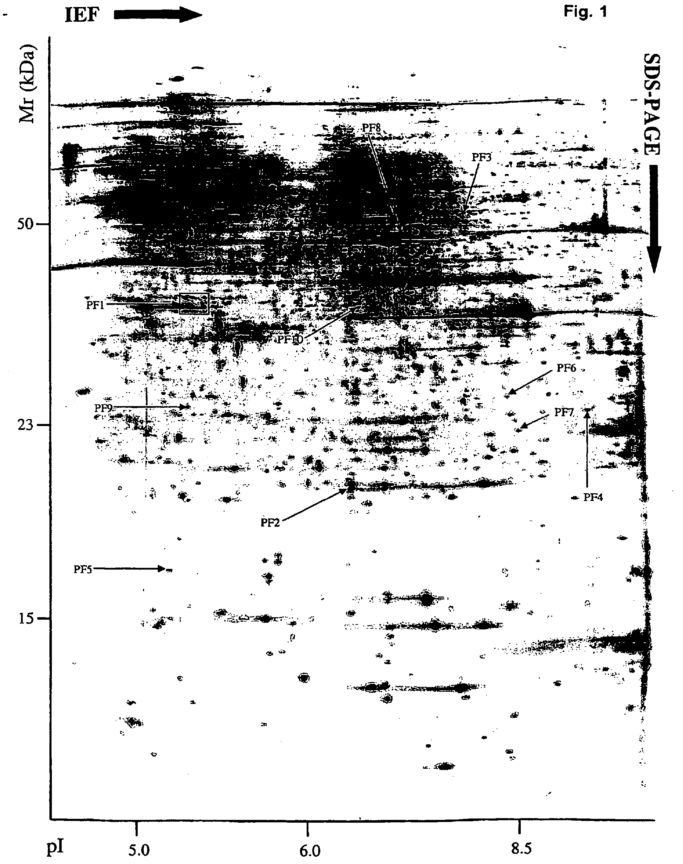

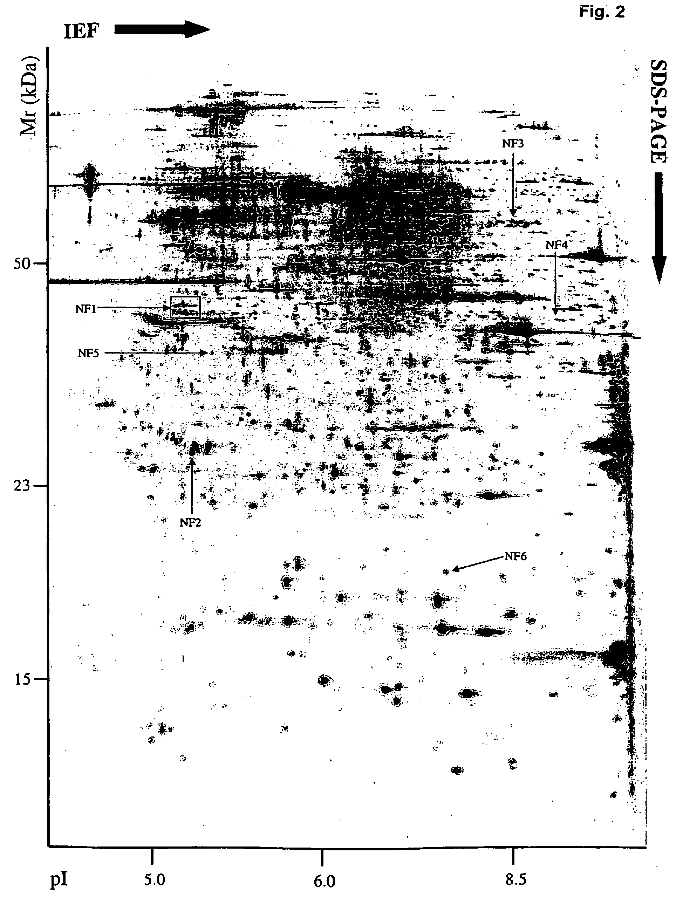

Apoptosis was induced in Jurkat T-cells by treatment with an anti-Fas antibody for six hours. 2-DE gels were produced after lysis of the cells and separation of the proteins. A representative 2-DE gel of Fas-induced Jurkat T-cells is shown in Figure 1. Approximately 2000 spots were resolved and detected by silver staining. Ten 2-DE gels of apoptotic cells were compared with ten 2-DE gels of Jurkat T-cells (Figure 2). Protein patterns of apoptosis-induced cells and control cells were found to be highly reproducible. In Fas-induced Jurkat T-cells 24 additional spots and in untreated Jurkat T-cells 21 additional spots were observed. Coomassie stained 2-DE gels were used for the identification by mass spectrometry.

### 2_{.}2 Identified proteins

The proteins of the total cell lysate (Table 1 a and Table 3) were identified within 21 spots by peptide mass fingerprinting after in-gel digestion with trypsin, elution of the generated peptides and analysis by DE-MALDI-MS (Figure 1 and 2). In the total cell lysate, 10 additional proteins were identified after Fas induction, whereas 6 proteins disappeared (Table 3). Four proteins (hnRNP A2/B1, hnRNP C1/C2, p54nrb and Rho GDI 2) were found at different spot positions in negative- and positive Fas cells, whereas the other proteins were only identified at one condition.

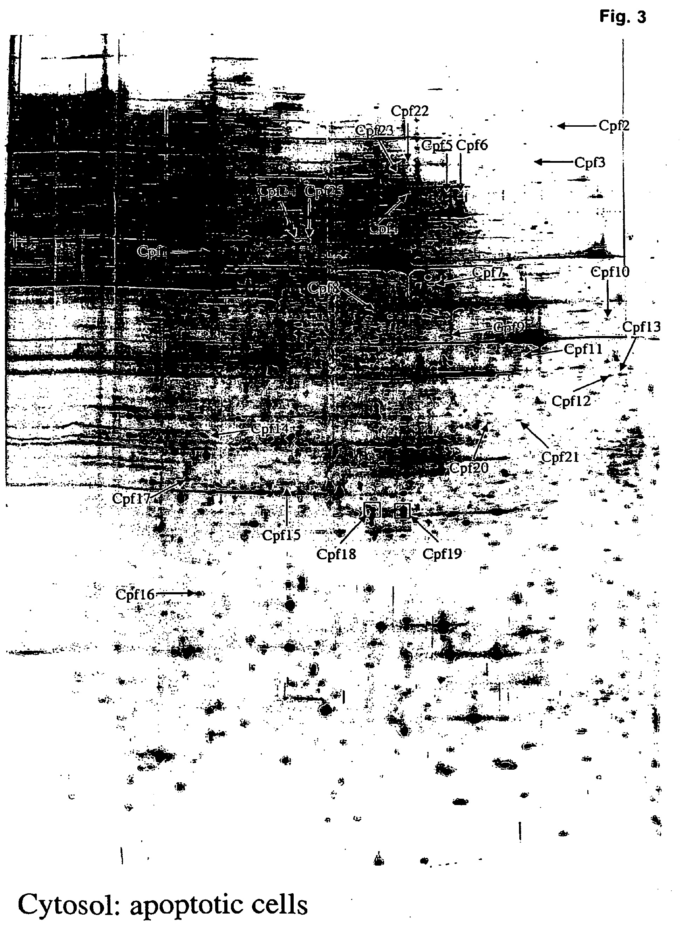

The molecular mass of protein spots in 2-DE gels can usually be determined with an accuracy of about 10%. The identified proteins in negative Fas gels displayed the theoretical mass of the corresponding protein. Five of the apoptosis-modified positive Fas proteins showed a significant decreased mass, whereas the remaining three proteins hnRNP C1/C2, p54nrb and splicing factor SRp30c retained the expected theoretical mass. The negative Fas spot of p54nrb showed an increased mass of 3.6 kD in comparison to the positive Fas spot of the same protein (Figure 3). The negative Fas spot of the hnRNP C1/C2 spots displayed an increased mass of 1 kD and decreased µl of 0.4 in comparison to the positve Fas spots. The mass and µl of the splicing factor SRp30c in Fas-positive Jurkat T-cells showed the theoretical values. These results indicate that predominantly cleavage events have occurred within the identified proteins during the apoptotic process.

The identified protein share similarities concerning function and motifs. The hnRNPs and the splicing factors are involved in the splicing process. 8 proteins contain the RNP-motif and 7 proteins include an aspartic acid/glutamic acid rich domain. Interaction with protein kinase CK2 was already identified for hnRNP A2/B1, hnRNP C1/C2, nucleolin and the transcription factor BTF3.

### 2.3 Prediction of cleavage sites after Fas-induction

Seven proteins were reduced in mass after Fas-induction. Considering the sequence coverage of the peptide mass fingerprint and the difference of the theoretical and the detected mass and µl lead to calculate approximately the cleavage site of the protein (Table 2). The identified protein spots of hnRNP A2/B1 and Rho GDI 2 was cleaved at the amino-terminal end, hnRNP A1, hnRNP R and p54nrb at the carboxy-terminal end and nucleolin at both sites.

The cleavage sites can be estimated more precisely by taking in account that caspases were responsible for the degradation. These enzymes cleave target proteins at specific aspartic acids. Only one cleavage site is possible for p54nrb, Rho GDI 2 and the amino-terminal cleavage of nucleolin, whereas two sites can be calculated for hnRNP A1, hnRNP A2/B1, hnRNP C1/C2 and for the carboxy-terminal cleavage of nucleolin (Table 2).

Concerning the specificities of the caspases, the most likely cleavage site for hnRNP A2/B1 is the sequence AEVD, for the carboxy-terminal cleavage of nucleolin the sequence AMED. The two possible cleavage sites of hnRNP A1 are quite equal concerning caspase specificity. Two cleavage sites can be calculated for hnRNP C1/C2 but it can be assumed likewise that the known phosphorylation may be the reason for the shift in µl, which is supported by the fact that hnRNP C1/C2 was identified in neighboring seven spots. The possible cleavage of hnRNP R was relatively difficult to calculate. Most reasonable was an amino- and carboxy-terminal cleavage which lead approximately to the found mass and pl.

The RNP consensus sequence of the RNP motif is composed of two short sequences, RNP1 and RNP2, and a number of other conserved amino acids (29). Five of the six identified shortened proteins contain one or more RNP motifs. The RNP1 and RNP2 consensus sequences of hnRNP A1, hnRNP R, p54nrb, one of the two of hnRNP A2/B1 and two of the four of nucleolin are within the sequence of the identified protein spots. No cleavage within the sequence from RNP2 to RNP1 has occurred. On the hand, the carboxy-terminal sequence in hnRNP A1, termed M9, was separated from the protein.

### 2.4 Results for cell compartments

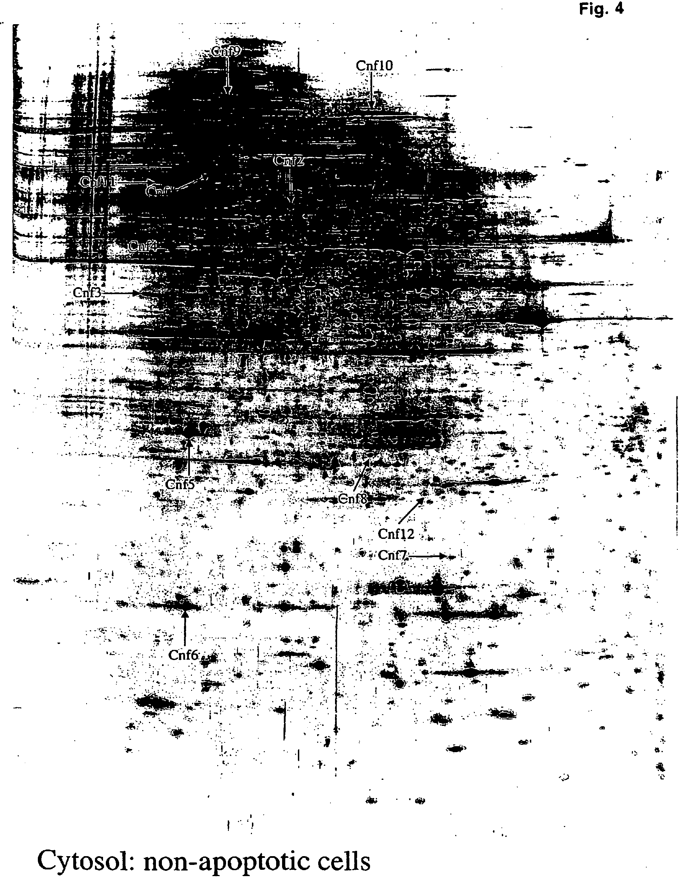

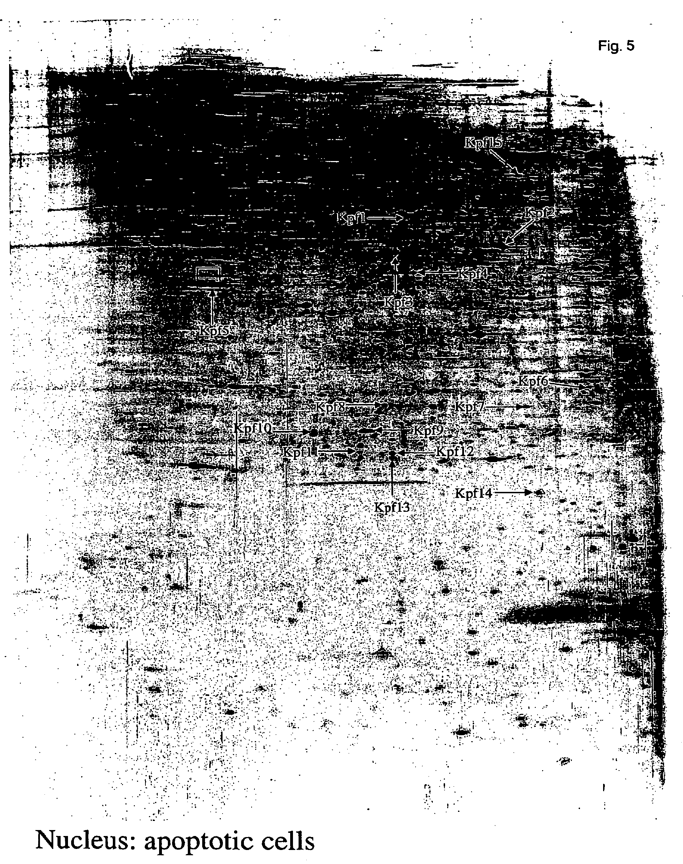

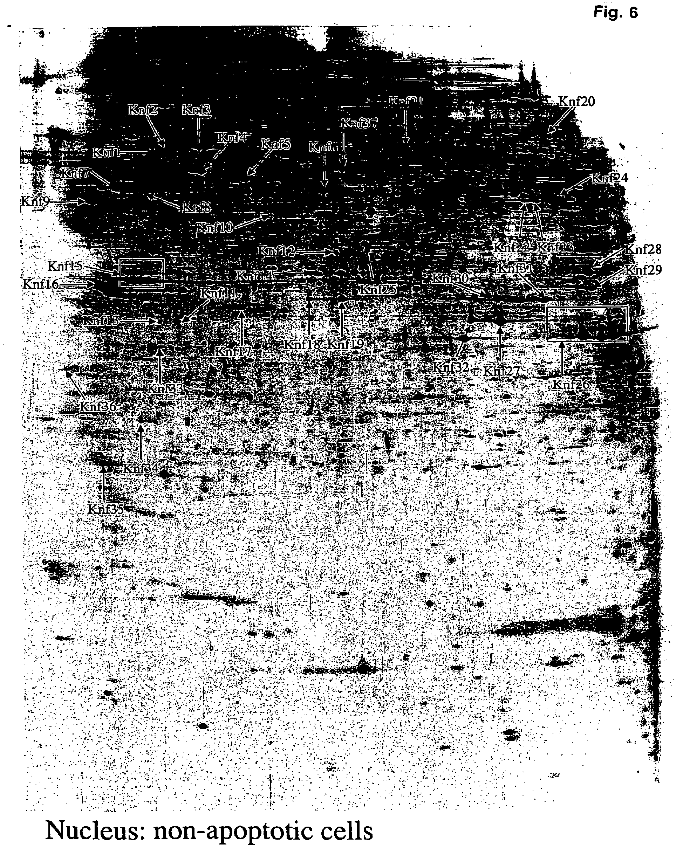

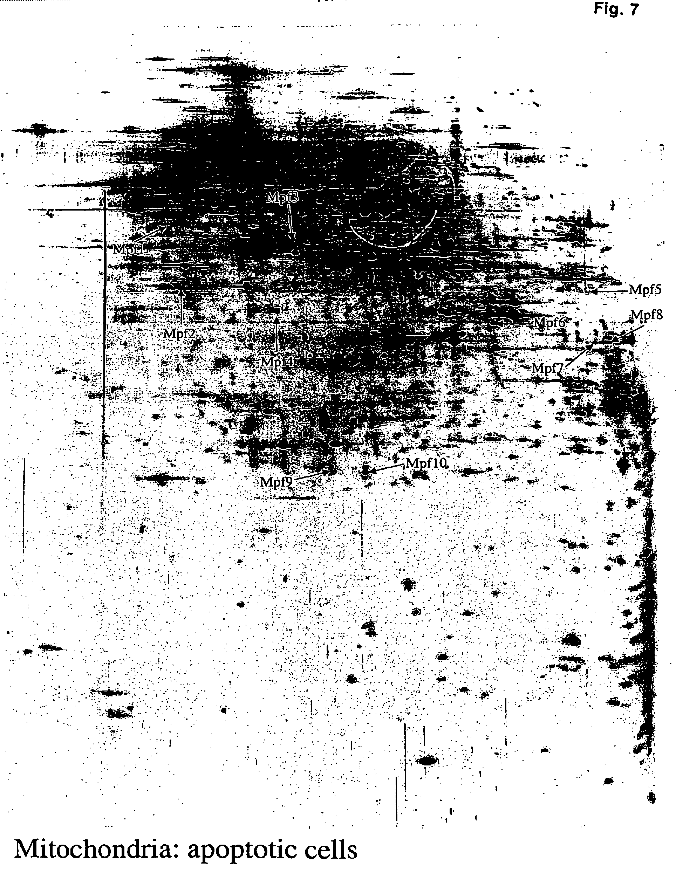

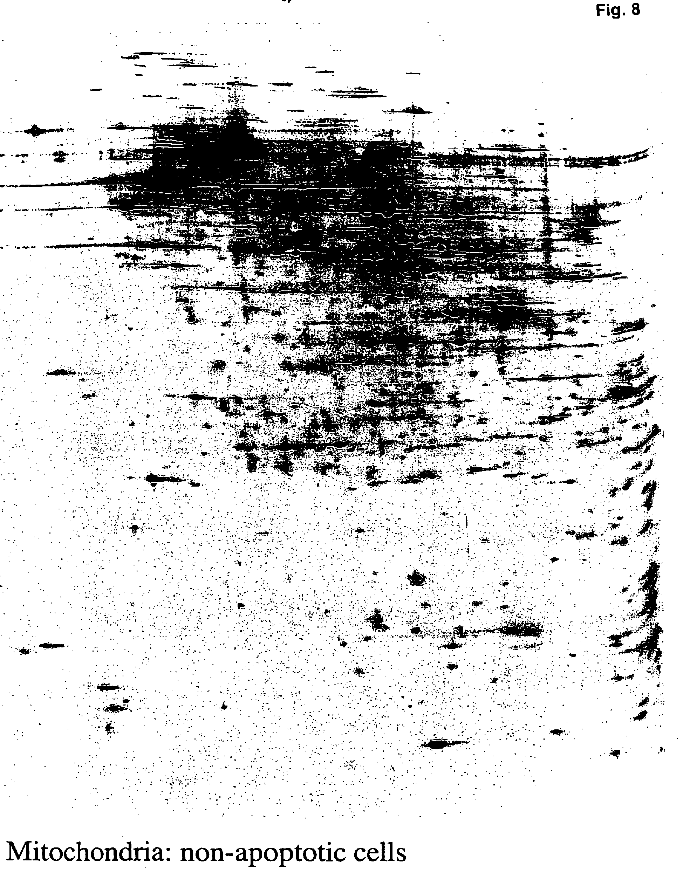

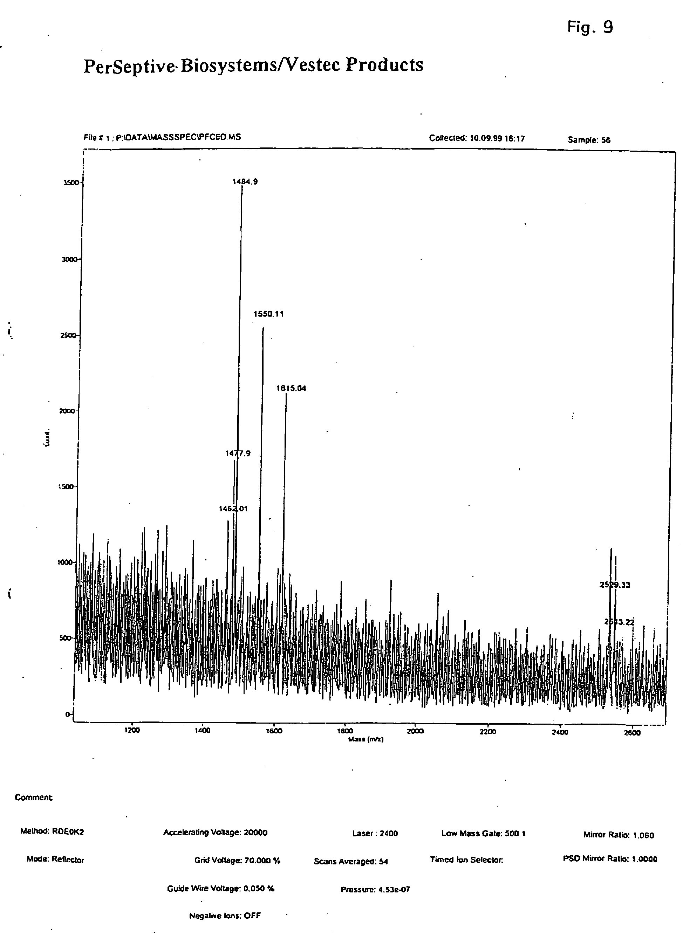

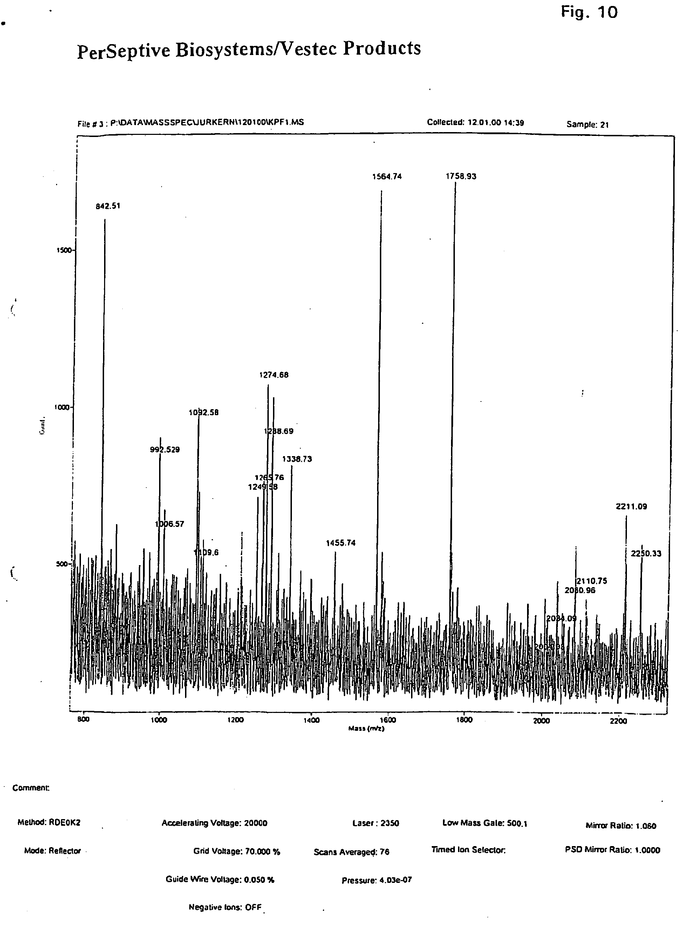

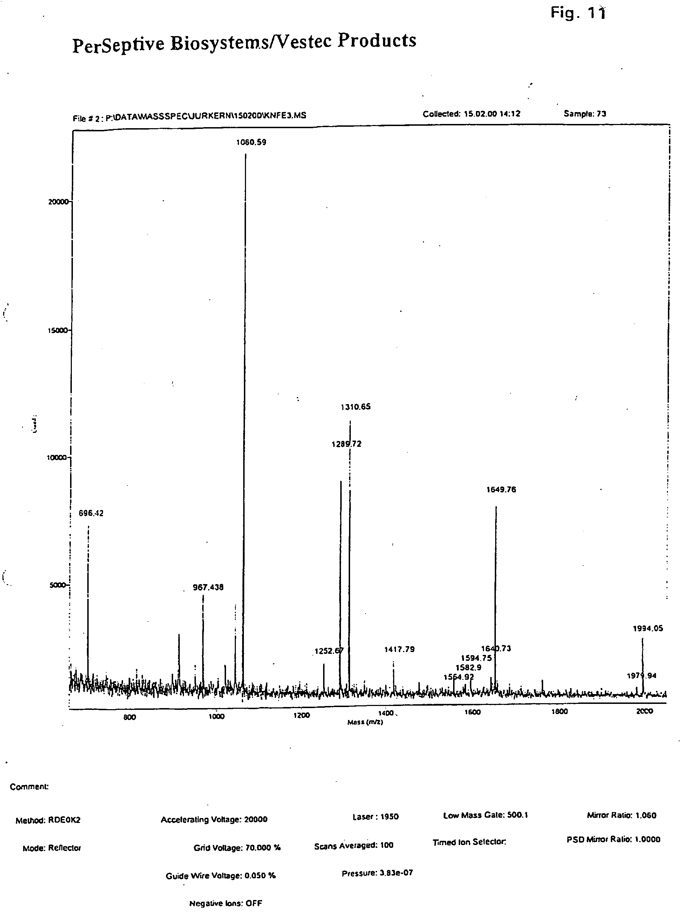

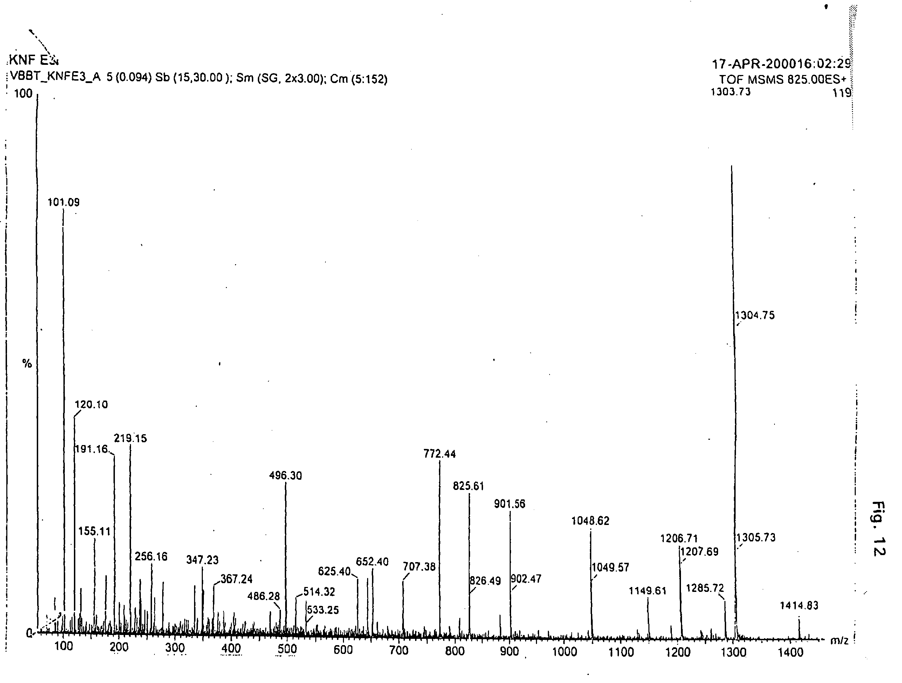

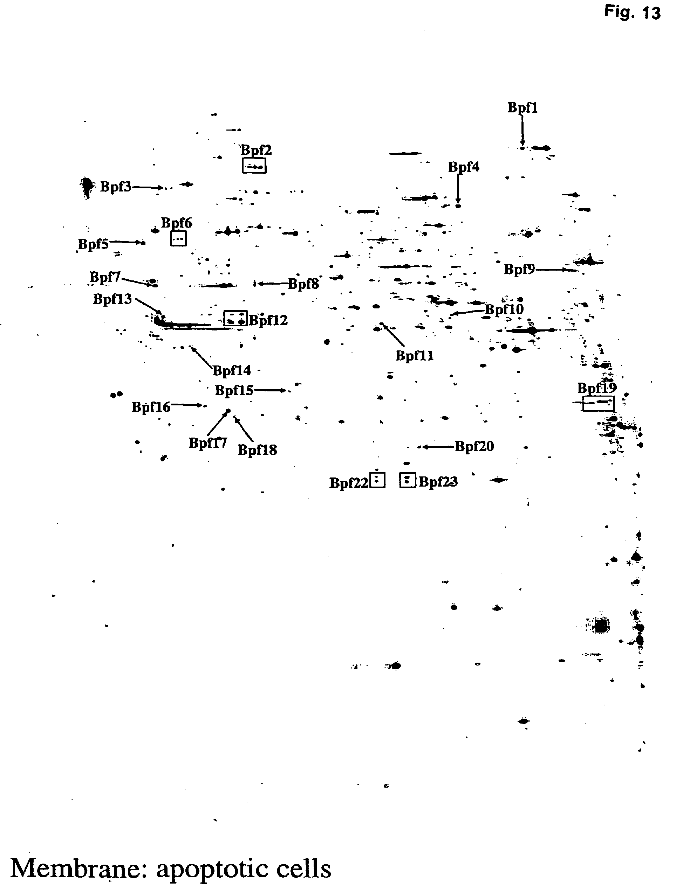

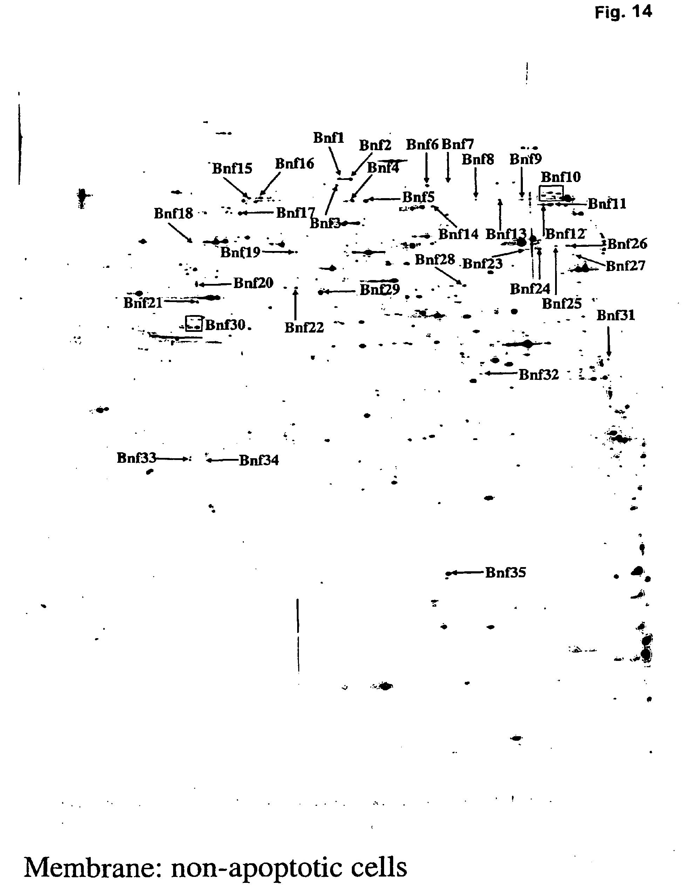

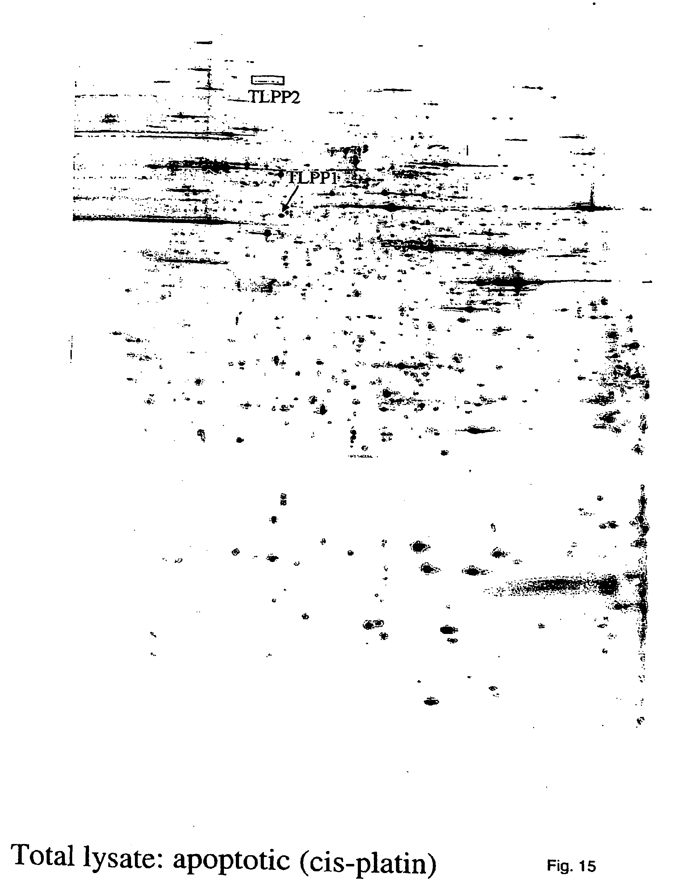

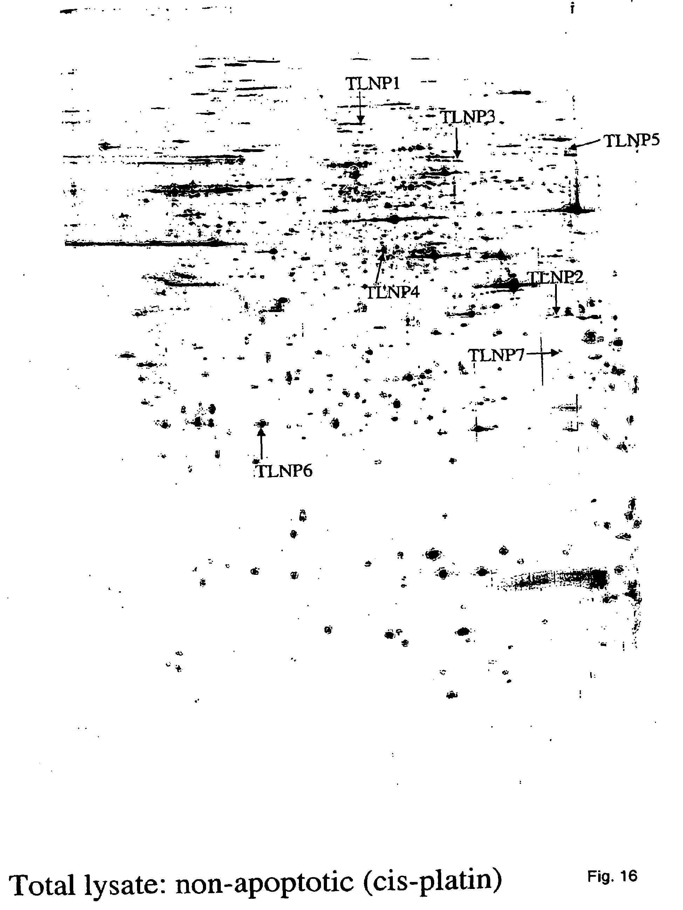

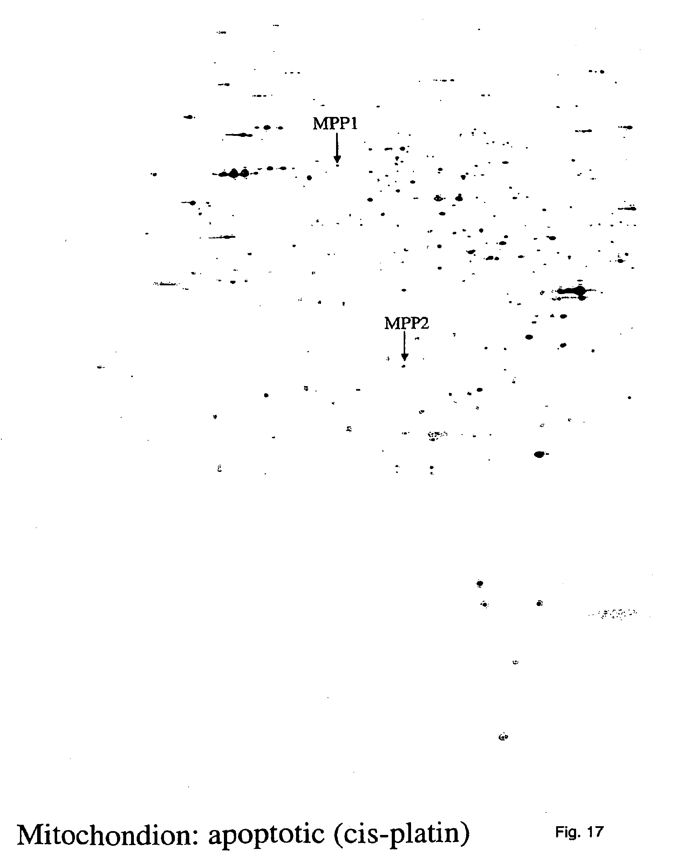

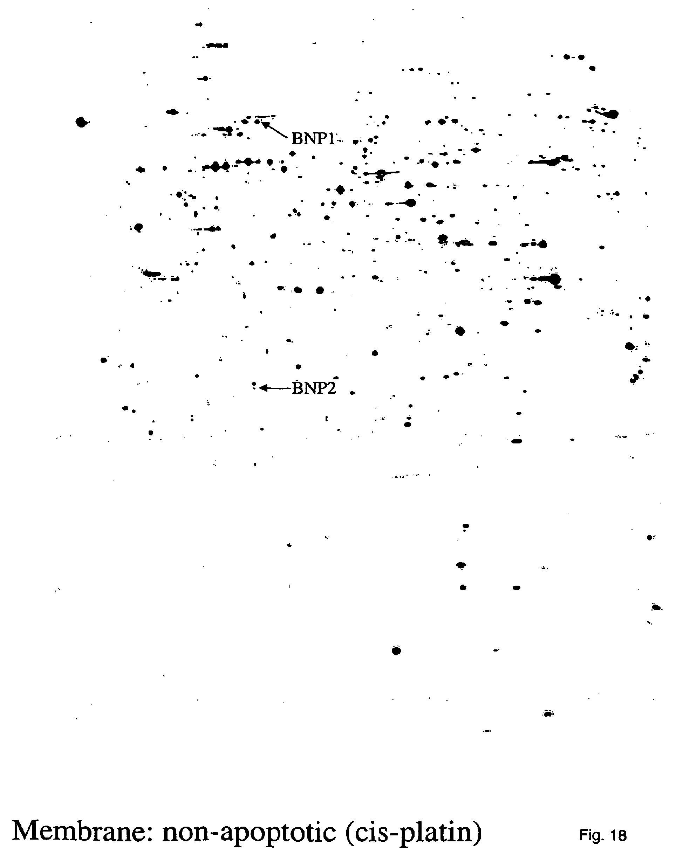

In addition to the total cell lysate, the cytosolic compartment, the nucleus, the mitochondria and the membrane were analysed. Since de novo synthesis of proteins was suppressed, the appearance or disappearance of proteins in cellular compartments after apoptosis induction indicates translocation of these proteins from one compartment to the other (e.g. 60 S ribosomal protein PO, Baf-57, Caf-1, FUSE binding protein 1, GAP SH3 binding protein, HDGF, HnRNP A/B, HnRNP A1, HnRNP A2/B1, HnRNP A3, HnRNP C1/C2, HnRNP D, HnRNP K, KH-type splicing regulatory protein, lamin B1, lamin B2, p54nrb, Rho GDI 2, Tat binding protein 1). After Fas induction, 25 additional proteins could be identified in the cytosol, whereas 12 proteins disappeared (Table 4, Fig. 3 and 4). In the nucleus, 15 additional proteins could be identified after Fas induction, whereas 37 disappeared (Table 5, Fig. 5 and 6). In the mitochondria, 10 additional proteins could be identified after Fas induction (Table 6, Fig. 7 and 8). In the membrane, 22 additional proteins could be identified after Fas induction, whereas 35 disappeared (Table 7, Fig. 13 and 14). After cis-platin induction, two additional proteins appeared in the total cell lysate, whereas seven proteins disappeared. In the membrane, two additional proteins appeared after apoptosis induction. In the mitochondria, two proteins disappeared (Table 8, Fig. 15, 16, 17, 18).

### 3. Discussion

Apoptosis-modified proteins were identified by a proteome approach after Fas-induction. The proteins which were found in the total cell lysate hnRNP A2/B1, hnRNP R, p54nrb, splicing factor ASF-2 and splicing factor SRp30c were not yet described to be related to apoptosis. The five proteins hnRNP A1, hnRNP C1/C2, nucleolin, Rho GDI 2 and transcription factor BTF3 were already known to be associated to apoptosis. These proteins were identified as well by a proteome approach in the human Burkitt Lymphoma cell line HL60 after IgM-mediated apoptosis (18,30,31). However, hnRNP A1, nucleolin and Rho GDI 2 were identified at other spot positions compared to the Jurkat T-cells. These results prove that the proteome approach can be useful to identify apoptosis-modifiecf proteins at different experimental conditions.

Separation of cellular compartments led to a significant increase of the sensitivity of protein detection and identification. In addition the translocation of proteins during apoptosis can be monitored in a highly sensitive way. Protein translocation plays a major role in apoptosis signalling. For example, apoptosis-inducing proteins are released from the mitochondria into the cytosol. Caspase activated DNAse (CAD) translocates from the cytosol to the nucleus. Interference with protein translocation might be a useful approach to modify the apoptosis process. Thus modulating protein translocation offers therapeutic possibilities in both, proliferative diseases with the aim to induce apoptosis as well as degenerative diseases with the aim to prevent apoptosis.

More than 60 substrates for caspases have been already described (6). These proteins can be activated or inactivated due to the cleavage. The caspase substrates are involved in different processes e.g. cell cycle, replication, transcription, translation, DNA cleavage, DNA repair and function as kinases, cytoskeletal and structural proteins. The results of this study indicated that cleavage events have occurred within the identified proteins, probably by caspases.

The most striking feature of the identified apoptosis-modified proteins of the total cell lysate is that eight of the proteins contain the RNP-binding motif and seven of the eight proteins, with the exception of nucleolin, are involved in the splicing process.

The RNP-motif, also known as RBD or RRM (29), was identified in about 300 proteins. It is composed of two consensus sequences, RNP2 and RNP1, and a number of other amino acids within a total length of about 90 amino acids. The three dimensional structure was solved first in the U1A spliceosomal protein. RNA-binding proteins are involved in the regulation of gene expression. In particular, the regulation of RNA by signalling allows a cell to respond much faster to a stimuli than protein expression from *de novo* transcription. Specific mRNAs can be stored as mRNA-protein complexes and in response to a stimulus the masking proteins are removed or modified and the mRNA is translated. Consideration of the identified protein spots revealed that no cleavage occurred within the RNP-motif. Hence it can be assumed that the RNA-binding properties are probably not affected by the apoptotic process.

Many proteins involved in alternative splicing contain RNA-protein binding motifs. Alternative splicing of pre-mRNA is a process for generating functionally different proteins from the same gene. The splicing reaction is catalyzed by the spliceosome, which is formed by small nuclear ribnucleoproteins (snRNPs) and a large number of splicing factors. In particular, proteins of the SR family play important roles in splicing control. Furhermore, phosphorylation modulates protein-protein interactions within the spliceosome.

An important factor for the complex regulation of apoptosis may well be pre-mRNA splicing. Alternative splicing was identified for some contributors to apoptosis. Death receptors, Bcl-2 family members, caspases and CED-4 showed alternative splice forms (32). Apoptosis-associated proteins can be generated by splicing with different functions and subcellular localization. The potential crucial role in regulation of apoptosis by splicing was confirmed strongly by the fact that the predominantly number and significance of the altered proteins were involved in splicing process.

### Reference List

1. Thompson, C.B. (1995) Science 267, 1456-1462
2. Nicholson, D.W. (1996) Nat.Biotechnol. 14, 297-301
3. Cohen, G.M. (1997) Biochem.J. 326, 1-16
4. Thornberry, N.A. and Lazebnik, Y. (1998) Science 281, 1312-1316
5. Miller, L.K. (1999) Trends Cell Biol. 9, 323-328
6. Stroh, C. and Schulze-Osthoff, K. (1998) Cell Death Differ. 5, 997-1000
7. Adams, J.M. and Cory, S. (1998) Science 281, 1322-1326
8. Gross, A., McDonnell, J.M., and Korsmeyer, S.J. (1999) Genes Dev. 13, 1899-1911
9. Ashkenazi, A. and Dixit, V.M. (1999) Curr.Opin.Cell Biol. 11, 255-260
10. Green, D. and Kroemer, G. (1998) Trends Cell Biol. 8, 267-271
11. Green, D.R. and Reed, J.C. (1998) Science 281, 1309-1312
12. Guo, M. and Hay.B.A. (1999) Curr. Opin. Cell Biol. 11, 745-752
13. Downward, J. (1998) Curr.Opin. Genet. Dev. 8, 49-54
14. Griffith, T.S. and Ferguson, T.A. (1997) Immunol. Today 18, 240-244
15. Nagata, S. and Golstein, P. (1995) Science 267, 1449-1456
16. O'Connell, J., Bennett, M.W., O'Sullivan, G.C., Collins, J.K., and Shanahan, F. (1999) Immunol. Today 20, 46-52
17. Pitti, R.M., Marsters, S.A., Lawrence, D.A., Roy, M., Kischkel, F.C., Dowd, P., Huang, A., Donahue, C.J., Sherwood, S.W., Baldwin, D.T., Godowski, P.J., Wood, W.I., Gurney, A.L., Hillan, K.J., Cohen, R.L., Goddard, A.D., Botstein, D., and Ashkenazi, A. (1998) Nature 396, 699-703
18. Brockstedt, E., Rickers, A., Kostka, S., Laubersheimer, A., Dorken, B., Wittmann-Liebold, B., Bommert, K., and Otto, A. (1998) J. Biol. Chem. 273, 28057-28064
19. Wilkins, M.R., Pasquali, C., Appel, R.D., Ou, K., Golaz, O., Sanchez, J.C., Yan, J.X., Gooley, A.A., Hughes, G., Humphery-Smith, I., Williams, K.L., and Hochstrasser, D.F. (1996) Biotechnology (N.Y.) 14, 61-65
20. O' Farrell, P.H. (1975) J. Biol. Chem. 250, 4007-4021
21. Klose, J. (1975) Humangenetik 26, 231-243
22. Aebersold, R. and Leavitt, J. (1990) Electrophoresis 11, 517-527
23. Fenn, J.B., Mann, M., Meng, C.K., Wong, S.F., and Whitehouse, C.M. (1989) Science 246, 64-71
24. Karas, M. and Hillenkamp, F. (1988) Anal.Chem. 60, 2299-2301
25. Appel, R.D., Bairoch, A., Sanchez, J.C., Vargas, J.R., Golaz, O., Pasquali, C., and Hochstrasser, D.F. (1996) Electrophoresis 17, 540-546
26. Lemkin, P.F. (1997) Electrophoresis 18, 461-470
27. Celis, J.E., Ostergaard, M., Jensen, N.A., Gromova, I., Rasmussen, H.H., and Gromov, P. (1998) FEBS Lett. 430, 64-72
28. Klose, J. and Kobalz, U. (1995) Electrophoresis 16, 1034-1059
29. Burd, C.G. and Dreyfuss, G. (1994) Science 265, 615-621
30. Brockstedt, E., Otto, A., Rickers, A., Bommert, K., and Wittmann-Liebold, B. (1999) J.Protein Chem. 18, 225-231
31. Rickers, A., Brockstedt, E., Mapara, M.Y., Otto, A., Dorken, B., and Bommert, K. (1998) Eur.J.Immunol. 28, 296-304
32. Jiang, Z.H. and Wu, J.Y. (1999) Proc.Soc.Exp.Biol.Med. 220, 64-72
33. Enari M, Sakahira H, Yokoyama H, Okawa K, Iwamatsu A, Nagata S: A caspase-activated DNAse that degrades DNA during apoptosis, and its inhibitor ICAD (1998) Nature 393, 393-396.
34. Yan et al. (1999) J. Biol. Chem. 274, 2145-2156.

**Table 1a**

| Table 1 a shows several identified apoptosis-modified proteins in Jurkat T-cells. Apoptosis was induced by Fas. | | | | | | | | | |
|---|---|---|---|---|---|---|---|---|---|
| Protein | NCBI | Spot | Mr found | Mr theor. | pl found | pl theor. | RNP- motif | D/E- rich | CK2' |
| hnRNP A1 | 133254 | PF6 | 32100 | 38846 | 8.5 | 9.26 | 2 | - | - |
| hnRNP A2/B1 | 4504447/ 133257 | NF4PF4 | 36400 29900 | 36006 / 37429 | 9.0 9.3 | 8.67/ 8.97 | 2 | - | + |
| hnRNP C1/C2' | 133298 | NF1 PF1 | 36300 35300 | 33298 | 5.0 5.4 | 5.11 | 1 | + | + |
| hnRNP R | 2697103 | PF8 | 49100 | 70943 | 7.3 | 8.23 | 1 | - | - |
| Nucleolin | 4885511 | PF5 | 18100 | 76344 | 5.2 | 4.59 | 4 | + | + |
| p54nrb | 1895081 | NF3 PF3 | 55900 52300 | 54231 | 8.5 8.1 | 9.01 | 2 | + | - |
| Rho GDI2 | 1707893 | NF2 PF2 | 23100 22100 | 22988 | 5.1 6.2 | 5.10 | - | + | |
| Splicing factor ASF-2 | 105294 | NF5 | 31400 | 31999 | 5.2 | 5.61 | 1 | + | - |
| Splicing factor SRp30c | 4506903 | PF7 | 27300 | 25542 | 8.6 | 8.70 | 1 | + | - |
| Transcription factor 8TF3 | 29507 | NF6 | 19000 | 17699 | 7.7 | 6.85 | - | + | + |

Known interactions with protein kinase CK2 are displayed with an asterisk *. The sign^{#} indicates that hnRNP C1/C2 was identified in seven spots, three times in negative Fas and four times in positive Fas Jurkat T- cells.

**Table 1b**

| Summary of factors modified by apoptosis. Detailed characterization is given in Tables 2-8 (T = total lysate, M = mitochondria, N = nucleus, C = cytosol, B = membrane). If nothing else is mentioned, apoptosis was induced by Fas. | | | | |
|---|---|---|---|---|
| Group | Proteins | Localization | Modification during apoptosis | known function |
| hnRNPs | A/B | NC | | RNP motif |
| | A1 | TMNC | | known substrate. RNP motif |
| | A2/B1 | TMNC | | RNP motif |
| | A3 | N | | RNP motif |
| | C1/C2 | TMN | | known substrate. RNP motif |
| | D | NC | | RNP motif |
| | F | M | | RNP motif |
| | H | NC | | RNP motif |
| | I | N | | RNP motif |
| | K | NC | | RNP motif |
| | L | N | | RNP motif |
| | R | TN | | RNP motif |
| | JKTBP1 | N | | RNP motif |
| Splicing factors | SRp30c | TN | only in apoptotic cells: also in the nucleus | splicing factor. RNP motif |
| | P54nrb | TN | modified; processed | splicing factor or nuclear matrix protein, respectively, RNP motif |
| | SF2p33 (ASF-2) | TN | missing in the nucleus of apoptotic cells | splicing factor; for after-native splicing variant function in the splicing of the Caspase-2 was described. RNP motif |
| | SF SC35 | N | missing in the nucleus of apoptotic cells; extreme IP shift | splicing factor; for alter-native splicing variant function in the splicing of the Caspase-2 was described. RNP motif |
| | NMP200 (rel. to SF PRP19) | N | missing in the nucleus of apoptotic cells | protein of the nuclear matrix; unknown function |
| | PTB-associated SF | C | Cytosol of apoptotic cells | splicing factor, RNP motif |
| Translation | 60S acidic ribosomal protein | MN | ribosomal protein; normal cell in the nucleus/ER; apoptotic cells in mitochondria, altered IP; probably by phosphorylation | known substrate |
| | EF-Tu | N | mitochondrial protein: found in the nucleus of apoptotic cells | translation |
| | EF-1 beta | N | missing in the nucleus of apoptotic cells | no function known for apoptosis |
| | EIF-5A | C | missing in the cytosol of apoptotic cells | cellular target of HIV type 1 Rev binding factor |
| Structural | Lamin 81 | NC | nucleus of apoptotic cells and as fragment in the cytosol of apoptotic cells | known substrate |
| | Lamin B2 | NC | nucleus of apoptotic cells and cytosol of apoptotic cells | known substrate |
| | Vimentin | N | nucleus of apoptotic cells | known substrate |
| | Beta-Tubulin | C | cytosol of apoptotic cells | known substrate |
| Signal transduction proteins | GAP SH3 binding protein | T | Lysate of apoptotic cells, presumably processed | Ras-Oncogene signal protein. RNP motif |
| | Rho GD12 | TMNC | | known substrate, published |
| | cGMP-dependent protein kinase type I alpha | M | mitochondria of apoptotic cells; presumably processed | Ser/Thr kinase, unknown function |
| Chromatin | Nucleolin | TC | in the lysate of apoptotic cells, presumably processed | known substrate; multi-functional protein, chromatin structure. RNP motif |
| | Baf-57 | N | missing in the nucleus of apoptotic cells | regulator of the chromatin structure |
| | CAF-1 (RB b.p.) (WD-repeats) | N | missing in the nucleus of apoptotic cells | binds to retinoblastoma, patent on WD repeat applications |
| Transcription factor | BTF3 | TC | missing in the lysate of apoptotic cells | already published |
| | CBF-beta | N | in the nucleus of apopiotic cells | binds to enhancers of murine leukaemia virus, Polioma virus. TCR etc., known alteration in the case of acute myeloid leukaemia |
| Proteasome | 26S protease subunit 12 | N | in the nucleus of apoptotic cells | regulatory subunit of the proteasome |
| | proteasome subunit C8 | C | in the cytosol of apoptotic cells | regulatory subunit of the proteasome |
| | Tat binding protein-1 | C | missing in the cytosol of apoptotic cells | regulatory subunit of the proteasome: HIV type 1 Tat binding protein |
| Mitochondrial | Isocitrate dehydrogenase | N | in the nucleus of apoptotic cells: maybe shortened | citrate cycle |
| | AOP-1 | N | N-terminal shortened, in the nucleus of apoptotic cells | anti-oxidant |
| Miscellaneous | Nucleophosmin | N | missing in apoptotic cells | already published |
| | SYT interacting protein SIP | N | missing in apoptotic cells | unknown, altered in synovial sarcoma cells |
| | PA1-G | N | in the nucleus of apoptotic cells | acetylhydrolase |
| | CRHSP-24 | N | in the nucleus of apoptotic cells | substrate of calcineurin |
| | HCD2 | NC | in the nucleus of apoptotic cells, missing in the cytosol of apoptotic cells, maybe phosphorylated | interaction with amyloid ß-peptide; Alzheimer's disease |
| | GMP synthase | C | in the cytosol of apoptotic cells | synthesis of guanin nuclectides, particularly GTP |
| | FUSE binding protein 1 | C | in the cytosol of apoptotic cells | activation of the tarupstream element of c-myc |
| | HDGF | C | missing in the cytosol of apoptotic cells | hepatoma-derived growth factor, mitogenic activity for fibroblasts |

**Additional proteins**

| **Group** | **Proteins** | **Localization** | **Modification during apoptosis** | **Known function** |
|---|---|---|---|---|
| | | | | |
| ***hnRNPs*** | HnRNP A0 | B | missing in the membrane of apoptotic cells | RNP motif |
| | Apobec-1 interacting protein | B | membrane of apoptotic cells | RNP motif; interaction with apolipoprotein B |
| | | | | |
| ***Splicing factors*** | Splicing factor 1 | B | missing in the membrane of apoptotic cells | KH motif |
| | KH-type splicing regulatory protein | C B | missing in the membrane of apoptotic cells, shortened in the cytosol of apoptotoc cells | KH motif |
| | | | | |
| ***Translation*** | 40 S ribosomal protein SA | B | membrane of apoptotic cells | |
| | Elongation factor 1 -delta | B | membrane of apoptotic cells | |
| | Elongation initation factor 3, subunit 4 | B | missing in the membrane of apoptotic cells | RNP motif |
| | Poly(A)-binding protein, cytoplasmic 4 | B | missing in the membrane of apoptotic cells | RNP motif |
| ***Structural*** | Gamma actin | B | missing in the membrane of apoptotic cells | |
| | Myosin heavy chain | B | membrane of apoptotic cells | |
| | | | | |
| ***Signal transduction*** | GAP SH3-binding protein 2 | B | missing in the membrane of apoptotic cells | RNP motif |
| | Small G protein | C | missing in the cytosol of apoptotic cells | Plasma membrane-associated GTP binding protein |
| | | | | |
| ***Chromatin*** | KIAA1470 | B | membrane of apoptotic cells | Regulator of chromosome condensation (RCCI)-motif |
| | | | | |
| ***Mitochondrial*** | ATP synthase beta chain | B | missing in the membrane of apoptotic cells | |
| | ATP synthase D chain | B | missing in the membrane of apoptotic cells | |
| | | | | |
| ***Miscellaneous*** | Alpha NAC | B | membrane of apoptotic cells | Nascent-polypeptide-associated complex protein; transcriptional coactivator |
| | ARDH | B | membrane of apoptotic cells | N-temiinal acetyltransferase |
| | Cargo selection protein | B | missing in the membrane of apoptotic cells | Mannose 6-phosphate receptor binding protein |
| | DAZ associated protein 1 | B | missing in the membrane of apoptotic cells | RNP motif |
| | DEAD box protein retinoblastoma | C | cytosol of apoptotic cells | |
| | Dihydrofolate reductase | C | missing in the cytosol of apoptotic cells | |
| | Hydroxyacyl-CoA | B | missing in the membrane | Trifunctional protein |
| | dehydrogenase/3-ketoacyl-CoA thiolase/enoyl-CoA hydratase | | of apoptoric cells | |
| | ER-60 | B | missing in the membrane of apoptotic cells | Disulfide isomerase, thioredoxin domains |
| | HCA56 | B | missing in the membrane of apoptoric cells | Hepatocellular carcinoma-associated antigen |
| | Hsp-105 | C | missing in the cytosol of apoptotic cells | Heat shock protein |
| | IGF-II mRNA-binding protein 1 | B | missing in the membrane of apoptotic cells | RNP motif, KH motif; Insulin-like growth factor mRNA-binding |
| | IGF-II mRNA-binding protein 3 | B | missing in the membrane of apoptotic cells | RNP motif, KH motif; Insulin-like growth factor mRNA-binding |
| | Lactate dehydrogenase A | B | missing in the membrane of apoptotic cells | |
| | NS-associated protein | C B | missing in the membrane of apoptotic cells, shortenedin the cytosol of apoptotic cells | RNP motif |
| | RAD 21 | B | membrane of apoptotic cells | DNA double-strand break repair |
| | RAD 23 homolog B | C | missing in the cytosol of apoptotic cells | DNA excision repair |
| | T-complex protein 1 beta subunit | B | membrane of apoptotic cells | |
| | Thioredoxin like protein | B | membrane of apoptotic cells | |
| | Unnamed protein | C | missing in the cytosol of apoptotic cells | |

| | | | | |
|---|---|---|---|---|
| T = Total lysate M = Mitochondria N = Nucleus C = Cytosol B = Membrane | | | | |

**Additional proteins, cis-platin induced**

| **Group** | **Proteins** | **Localization** | **Modification during apoptosis** | **Known function** |
|---|---|---|---|---|
| | | | | |
| ***hnRNPs*** | HnRNP E1- | TL | missing in the lysate of apoptotic cells | KH-motiv |
| | HnRNP M | TL | missing in the lysate of apoptotic cells | RNP-motiv |
| | | | | |
| ***Structural*** | Alpha-Fodrin | TL | in the lysate of apop cells | |
| | | | | |
| ***Proteasome*** | 26 S protease subunit 4 | M | mitochondria of apoptotic cells | |
| | Proteasome subunit alpha type I | M | mitochondria of apoptotic cells | |
| | | | | |
| ***Miscellaneous*** | Chondrosarcomaassociated protein 2 | TL | missing in the lysate of apoptotic cells | |
| | ELAV-like 1 (Hu antigen R) | TL | missing in the lysate of apoptotic cells | RNP-motiv |
| | Glutathione S-transferase | TL | missing in the lysate of apoptotic cells | |
| | Hsp-60 | TL | in the lysate of apop cells | Chaperone |
| | Mortalin-2 (Heat shock 70kd protein 9B) | B | membrane of apopototic cells | Chaperone |
| | Prohibitin | B | membrane of apopototic cells | Inhibitor of DNA synthesis |
| | SKI interacting protein | TL | missing in the lysate of apoptotic cells | |
| | VDAC 3 | TL | missing in the lysate of apoptotic cells | Ion channel |

| | | | | |
|---|---|---|---|---|
| TL = Total lysate B = Membrane M = Mitochondria | | | | |

**Table 2**

| Prediction of cleavage sites for apoptosis-modified proteins found in the total cell lysate | | | | | | | | | |
|---|---|---|---|---|---|---|---|---|---|
| Protein | No. AA | Sequence coverage | Cleavage site | Start-end AA | Mass (kDa) | Mass found | pl | pl found | Putative cleaved sequence |
| hnRNP A1 | 371 | 15-178 | CT | 1-288 | 30.5 | 32.1 | 8.4 | 8.5 | GSYD |
| | | | | 1-314 | 32.9 | | 8.4 | | SYND |
| hnRNP | 351 | 102-350 | NT* | 49-353 | 31.6 | 29.9 | 8.9 | 9.3 | KLTD |
| A2/B1 | | | | 56-353 | 30.8 | | 9.2 | | VMRD |
| | | | | 76-353 | 28.6 | | 8.8 | | AEVD |
| HnRNP | 303 | 18-151 | - | 1-295 | 32.5 | 35.3 | 5.2 | 5.4 | EGED |
| C1/C2 | | | | 10-303 | 32.3 | | 5.0 | | NKTD |
| hnRNP R | 624 | 134-441 | CT | 1-463 | 52.1 | 49.1 | 5.9 | 7.3 | YPPD |
| | | | | 20-463 | 49.9 | | 6.5 | | EPMD+ YPPD |
| Nucleo -lin | 706 | 458-624 | NT+CT | 454-628 | 19.4 | 18.1 | 5.0 | 5.2 | TEID + AMED |
| | | | | 454-632 | 19.8 | | 4.9 | | TEID + GEID |
| p54nrb | 471 | 76-336 | (CT*) | 1-422 | 49.2 | 52.3 | 8.4 | 8.1 | MMPD |
| Rho GDI 2 | 201 | 22-196 | NT | 22-196 | 20.9 | 22.1 | 6.2 | 6.2 | DELD |

| | | | | | | | | | |
|---|---|---|---|---|---|---|---|---|---|
| The asterisk * displays that the comparison of the PMF of negative- and positive Fas showed an additional intense peak of the negative Fas spot outside the covered sequence and confirms the cleavage site (Figure 3). In parenthesis means that the cleavage site could not clearly identified only by sequence coverage of the PMF of the positive Fas spot. | | | | | | | | | |

**Table 3**

| Table 3 shows proteins of the total cell lysate. Apoptosis was induced by Fas. | | | | | | |
|---|---|---|---|---|---|---|
| Spot | Protein | NCBI | Mr | Mr | pI | pI |
| | | | theor. | found | theor. | found |
| PFI | hnRNP C1/C2 | 4758544 | 31966 | 35300 | 5.10 | 5.3 |
| PF2 | RhoGDI 2 | 1707893 | 22988 | 22400 | 5.10 | 6.2 |
| PF3 | P54nrb | 1895081 | 54231 | 52300 | 9.01 | 8.1 |
| PF4 | hnRNP A2/µB1 | 4504447/ | 36006/ | 36300 | 8.67/8.97 | 9.6 |
| | | 133257 | | | | |
| | | | 37429 | | | |
| PF5 | Nucleolin | 4885511 | 76344 | 18100 | 4.59 | 5.2 |
| PF6 | hnRNP A1 | 133254 | 38846 | 35200 | 9.26 | 9.6 |
| PF7 | Splicing factor SRp30c | 4506903 | 25542 | 27300 | 8.70 | 8.6 |
| PF8 | hnRNP R | 2697103 | 70943 | 49100 | 8.23 | 7.3 |
| PF9 | Unknown¹ | = | = | 24900 | = | 5.3 |
| PF10. | GAP SH3 binding protein | 5031703 | 52164 | 37000 | 5,37 | 6,2 |
| NF1 | hnRNP C1/C2 | 4758544 | 31966 | 36300 | 5.10 | 5.3 |
| NF2 | RhoGDI 2 | 1707893 | 22988 | 22400 | 5.10 | 6.4 |
| NF3 | P54nrb | 1895081 | 54231 | 55900 | 9.01 | 8.5 |
| NF4 | hnRNP A2/B1 | 4504447/ | 36006/ | 35700 | 8.67/8.97 | 8.7 |
| | | 133257 | | | | |
| | | | 37429 | | | |
| NF5 | Splicing factor 2p33 (ASF-2) | 105294 | 31999 | 31400 | 5.61 | 5.2 |
| NF6 | Transcription factor BTF3 | 29507 | 17699 | 19000 | 6.85 | 7.7 |

| | | | | | | |
|---|---|---|---|---|---|---|
| ¹ : Peptide mass fingerprint of the tryptic digestion (internal name: PFC6D) | | | | | | |

**Table 4**

| Table 4 shows proteins of the cytosol. Apoptosis was induced by Fas. | | | | | | |
|---|---|---|---|---|---|---|
| Spot | Protein | NCBI | Mr | Mr | pl | pl |
| | | | | | | |
| | | | theor. | found | theor. | found |
| | | | | | | |
| Cpf1 | Beta-Tubulin | 135448 | 49759 | 49800 | 4.75 | 5.2 |
| Cpf2 | PTB-associated splicing factor | 4826998 | 76149 | 89000 | 9.45 | 8.7 |
| Cpf3 | PTB-associated splicing factor | 4826998 | 76149 | 76000 | 9.45 | 8.3 |
| | | | | | | |
| Cpf4 | GMP synthase | 4504035 | 76715 | 62100 | 6.42 | 6.9 |
| Cpf5 | FUSE binding protein 1 | 4503801 | 67534 | 65200 | 7.21 | 7.7 |
| Cpf6 | FUSE binding protein 1 | 4503801 | 67534 | 65400 | 7.21 | 7.8 |
| Cpf7 | hnRNP D | 870749 | 38434 | 43100 | 7.61 | 7.4 |
| Cpf8 | hnRNP A/B | 4758542 | 31233 | 39100 | 9.35 | 6.6 |
| Cpf9 | hnRNP A/B | 4758542 | 31233 | 36200 | 9.35 | 7.6 |
| Cpf10 | hnRNP A2/B1 | 4504447 | 36006/ | 39000 | 8.67/8.97 | 9.6 |
| | | /133257 | 37429 | | | |
| Cpf11 | hnRNP A2/B1 | 4504447 | 36006/ | 35700 | 8.67/8.97 | 8.4 |
| | | /133257 | 37429 | | | |
| Cpf12 | hnRNP A1 | 133254 | 38846 | 35200 | 9.26 | 9.5 |
| Cpf13 | hnRNP A1 | 133254 | 38846 | 35200 | 9.26 | 9.6 |
| Cpf14 | Proteasome subunit C8 | 130859 | 28433 | 26600 | 5.19 | 5.2 |
| Cpf15 | Lamin B2 | 547822 | 59001 | 22400 | 5.87 | 5.8 |
| Cpf16 | Nucleolin | 4885511 | 76344 | 19000 | 4.59 | 5.2 |
| Cpf17 | Lamin B1 | 5031877 | 66408 | 22400 | 5.11 | 5.0 |
| Cpf18 | RhoGDI 2 | 1707893 | 22988 | 22400 | 5.10 | 6.2 |
| Cpf19 | RhoGDI 2 | 1707893 | 22988 | 22400 | 5.10 | 6.4 |
| Cpf20 | hnRNP A1 | 133254 | 38846 | 32100 | 9.26 | 8.1 |
| Cpf21 | hnRNP A1 | 133254 | 38846 | 35200 | 9.26 | 9.6 |
| Cnf1 | hnRNP K | 631471 | 51072 | 65500 | 5.14 | 5.2 |
| Cnf2 | hnRNP H | 1710632 | 49229 | 54100 | 5.89 | 6.1 |
| Cnf3 | HDGF | 4758516 | 26788 | 36100 | 4.70 | 4.7 |
| Cnf4 | Tat binding protein-1 | 4506211 | 45165 | 45100 | 5.31 | 5.0 |
| Cnf5 | RhoGDI 2 | 1707893 | 22988 | 23100 | 5.10 | 5.1 |
| Cnf6 | EIF-5A | 4503545 | 16701 | 17000 | 5.08 | 5.3 |
| Conf7 | Transcription factor BTF3 | 29507 | 17699 | 19000 | 6.85 | 7.7 |
| Cnf8 | HCD2 | 4758504 | 26923 | 24800 | 7.65 | 6.2 |

**Table 5**

| Table 5 shows proteins of the nucleus. Apoptosis was induced by Fas. | | | | | | |
|---|---|---|---|---|---|---|
| Spot | Protein | NCBI | Mr | Mr | pI | pI |
| | | | | | | |
| | | | theor. | found | tbeor. | found |
| | | | | | | |
| Kpf1 | hnRNP R | 2697103 | 70943 | 49100 | 8.23 | 7.2 |
| Kpf2 | Isocitrate dehydrogenase | 4504575 | 46644 | 43100 | 8.32 | 8.2 |
| | | | | | | |
| Kpf3 | Elongation factor Tu | 4507733 | 49540 | 41400 | 7.26 | 7.0 |
| | | | | | | |
| Kpf4 | 26S proteasome regulatory chain 12 | 4506231 | 37060 | 38800 | 6.11 | 7.2 |
| Kpf5 | hnRNP C1/C2 | 4758544 | 31966 | 35300 | 5.10 | 5.3 |
| Kpf6 | hnRNP A2/B1 | 4504447/ | 36006/ | 30000 | 8.67/ | 9.0 |
| | | 133257 | 37429 | | 8.97 | |
| Kpf7 | Splicing factor SRp30c | 4506903 | 25542 | 28300 | 8.74 | 8.6 |
| Kpf8 | PA1-G | 4505587 | 25734 | 28500 | 6.33 | 6.3 |
| Kpf9 | HCD2 | 4758504 | 26923 | 24800 | 7.65 | 6.2 |
| Kpf10 | AOP-1 | 5802974 | 27692 | 22500 | 7.67 | 6.1 |
| Kpf11 | Rbo GDI 2 | 1707893 | 22988 | 22400 | 5.10 | 6.2 |
| Kpf12 | Rho GDI 2 | 1707893 | 22988 | 22400 | 5.10 | 6.4 |
| Kpf13 | Rho GDI 2 | 1707893 | 22988 | 22100 | 5.10 | 6.4 |
| | | 2498753 | 21508 | | | |
| | CBF-beta | | | | 6.23 | |
| Kpf14 | CRHSP-24 | 4583307 | 15934 | 21300 | 8.41 | 8.8 |
| Kpf15 | Unknown² | - | - | 59100 | - | 8.3 |
| Knf1 | hnRNP K | 631471 | 51072 | 65600 | 5.14 | 5.2 |
| Knf2 | Lamin B1 | 125953 | 66408 | 65600 | 5.11 | 5.2 |
| Knf3 | hnRNP K | 631471 | 51072 | 65100 | 5.14 | 5.4 |
| Knf4 | Lamin B2 | 547822 | 59001 | 62800 | 5.87 | 5.4 |
| Knf5 | hnRNP K | 631471 | 51072 | 59700 | 5.14 | 6.0 |
| Knf6 | NMP200 (related to splicing factor | 5689738 | 55181 | 55500 | 6.14 | 6.4 |
| | PRP19) | | | | | |
| Knf7 | BAF57 | 4507089 | 46649 | 54700 | 4.85 | 4.9 |
| Knf8 | Vimentin | 2119204 | 53651 | 56200 | 5.06 | 5.1 |
| Knf9 | CAF-1 | 422892 | 46158 | 53000 | 4.90 | 4.7 |
| Knf10 | hnRNP H | 1710632 | 49229 | 50600 | 5.89 | 6.1 |
| Knf11 | Splicing factor 2p33 (ASF-2) | 105294 | 31999 | 31400 | 5.61 | 5.2 |
| Knf12 | hnRNP H | 1710632 | 49229 | 42100 | 5.89 | 6.6 |
| Knf13 | Splicing factor 2p33 (ASF-2) | 105294 | 31999 | 31400 | 5.61 | 5.1 |
| Knf14 | hnRNP A/B | 4758542 | 31233 | 39000 | 9.35 | 6.4 |
| Knf15 | hnRNP C1/C2 | 4758544 | 31966 | 36300 | 5.10 | 5.0 |
| Knf16 | Nucleophosmin | 114762 | 32575 | 35300 | 4.64 | 4.8 |
| Knf17 | 60S acidic ribosomal protein | 4506667 | 34273 | 33500 | 5.72 | 5.8 |
| Knf18 | JKTBP1 | 2780748 | 33589 | 36100 | 6.85 | 6.3 |
| Knf19 | JKTBP1 | 2780748 | 33589 | 36100 | 6.85 | 6.6 |
| Knf20 | SYT interacting protein SIP | 5454064 | 69492 | 73000 | 9.68 | 9.0 |
| Knf21 | hnRNP L | 4557645 | 60187 | 67400 | 6.65 | 7.4 |
| Knf22 | hnRNP I | 131528 | 57221 | 53700 | 9.22 | 8.5 |
| Knf23 | hnRNP I | 131528 | 57221 | 53900 | 9.22 | 8.6 |
| Knf24 | P54nrb | 1895081 | 54231 | 54000 | 9.01 | 9.2 |
| Knf25 | hnRNP D | 870749 | 38434 | 44100 | 7.61 | 6.9 |
| Knf26 | hnRNP A1 | 133254 | 38846 | 35200 | 9.26 | 9.6 |
| Knf27 | hnRNP A2/B1 | 4504447/ | 36006/ | 35700 | 8.67/ | 8.4 |
| | | 133257 | 37429 | | 8.97 | |
| Knf28 | hnRNP A3 | 1710627 | 39686 | 39000 | 8.74 | 9.6 |
| Knf29 | hnRNP A2/B1 | 4504447/ | 36006/ | 36400 | 8.67/ | 9.7 |
| | | 133257 | 37429 | | 8.97 | |
| Knf30 | hnRNP A3 | 1710627 | 39686 | 36400 | 8.74 | 8.3 |
| Knf31 | hnRNP A2/B1 | 4504447/ | 36006/ | 36200 | 8.67/ | 8.9 |
| | | 133257 | 37429 | | 8.97 | |
| Knf32 | hnRNP A2/B1 | 4504447/ | 36006/ | 35700 | 8.67/ | 8.2 |
| | | 133257 | 37429 | | 8.97 | |
| Knf33 | Splicing factor SC35 | 539663 | 25476 | 28100 | 11.86 | 5.1 |
| Knf34 | RhoGDI 2 | 1707893 | 22988 | 23100 | 5.10 | 5.1 |
| Knf35 | RhoGDI 2 | 1707893 | 22988 | 21300 | 5.10 | 4.8 |
| Knf36 | Elongation factor 1-beta | 4503477 | 24763 | 24800 | 4.50 | 4.5 |
| Knf37 | Unknown¹ | - | - | 61300 | - | 6.6 |

| | | | | | | |
|---|---|---|---|---|---|---|
| ¹ : Peptide mass fingerprint of the tryptic digestion and MS/MS-spectrum of mass 1649,76 dalton with the sequence TPGT(F/Mox)E (internal name: KNFE3) ² : Peptide mass fingerprint of the tryptic digestion (internal name: KPF1) | | | | | | |

**Table 6**

| **Table 6 shows proteins of the mitochondria. Apoptosis was induced by Fas.** | | | | | | |
|---|---|---|---|---|---|---|
| Spot | Protein | NCBI | Mr | Mr | pI | pI |
| | | | | | | |
| | | | theor. | found | theor. | found |
| | | | | | | |
| Mpf1 | hnRNP F | 4836760 | 45672 | 47600 | 5.38 | 5.2 |
| Mpf2 | hnRNP C1/C2 | 4758544 | 31966 | 35300 | 5.10 | 5.3 |
| Mpf3 | CGMP-dependent protein kinase type I alpha | 6225588 | 76364 | 45300 | 5.74 | 6.2 |
| | | | | | | |
| | | 4504453 | 51072 | | 5.14 | |
| | hnRNP K | | | | | |
| Mpf4 | 60S acidic ribosomal protein | 4506667 | 34273 | 33300 | 5.72 | 6.1 |
| Mpf5 | hnRNP A2/B1 | 4504447/ | 36006/ | 36300 | 8.67/8.97 | 9.6 |
| | | 133257 | | | | |
| | | | 37429 | | | |
| Mpf6 | hnRNP A2/B1 | 4504447/ | 36006/ | 35700 | 8.67/8.97 | 8.7 |
| | | 133257 | | | | |
| | | | 37429 | | | |
| Mpf7 | hnRNP A1 | 133254 | 38846 | 35200 | 9.26 | 9.6 |
| Mpf8 | hnRNP A1 | 133254 | 38846 | 35200 | 9.26 | 9.7 |
| Mpf9 | RhoGDI 2 | 1707893 | 22988 | 22100 | 5.10 | 6.2 |
| Mpf10 | RhoGDI 2 | 1707893 | 22988 | 22100 | 5.10 | 6.4 |

**Table 7**

| **Table 7 shows proteins of the membrane. Apoptosis was induced by Fas.** | | | | | | |
|---|---|---|---|---|---|---|
| **Spot** | **Protein** | **NCBI** | **Mr theor.** | **Mr found** | **pI theor.** | **pI found** |
| Bpf1 | PTB associated splicing factor | 4826998 | 76150 | 89200 | 9.5 | 8.3 |
| Bpf2 | Myosin heavy chain, nonmuscle | 189036 | 145080 | 78500 | 5.2 | 5.4 |
| Bpf3 | Rad 21 | 1620398 | 71670 | 70200 | 4.5 | 4.9 |
| Bpf4 | Fuse binding protein I | 4503801 | 67530 | 65400 | 7.2 | 7.8 |
| Bpf5 | Caf-1 | 422892 | 46160 | 53000 | 4.9 | 4.7 |
| Bpf6 | Baf-57 Beta Tubulin | 4507089 135448 | 46650 49760 | 54700 | 4.9 4.7 | 4.9 |
| Bpf7 | 40 S ribosomal protein SA | 86715 | 31780 | 43000 | 4.8 | 4.8 |
| Bpf8 | Tat binding protein 1 | 4506211 | 45150 | 42900 | 5.3 | 5.4 |
| Bpf9 | KIAA1470 | 7959201 | 60460 | 46300 | 9.4 | 9.2 |
| Bpf10 | Apabec-1 interacting protein | 1814274 | 36590 | 38600 | 9.1 | 7.7 |
| Bpf11 | Gap SH3 binding protein | 5031703 | 52150 | 36300 | 5.4 | 6.2 |
| Bpf12 | HnRNP C1/C2 | 4758544 | 31950 | 35300 | 5.1 | 5.3 |
| Bpf13 | HDGF | 4758516 | 26770 | 36100 | 4.7 | 4.7 |
| Bpf14 | EF-I delta Thioredoxin-like protein | 461994 4759274 | 31220 32230 | 31800 | 5.0 4.8 | 5.0 |
| Bpf15 | ARDH | 728880 | 26440 | 24700 | 5.4 | 5.8 |
| Bpf16 | Alpha NAC | 5031931 | 23370 | 26800 | 4.5 | 5.0 |
| Bpf17 | Alpha NAC | 5031931 | 23370 | 26600 | 4.5 | 5.2 |
| Bpf18 | Alpha NAC | 5031931 | 23370 | 26600 | 4.5 | 5.2 |
| Bpf19 | HnRNP A2/BI | 4504447/ 133257 | 36000/ 37430 | 32400 | 8.7/ 8.9 | 9.5 |
| Bpf20 | T-complex protein 1 beta subunit | 1871210 | 22920 | 26700 | 5.9 | 6.7 |
| Bpf21 | RhoGDI 2 | 1707893 | 22970 | 22300 | 5.1 | 6.4 |
| Bpf22 | RhoGDI 2 | 1707893 | 22970 | 22300 | 5.1 | 6.2 |
| | | | | | | |
| Bnf1 | KH-type splicing regulatory protein | 4504865 | 73140 | 78900 | 6.9 | 6.4 |
| Bnf2 | KH-type splicing regulatory protein | 4504865 | 73140 | 78900 | 6.9 | 6.5 |
| Bnf3 | KH-type splicing regulatory protein | 4504865 | 73140 | 76600 | 6.9 | 6.4 |
| Bnf4 | FUSE binding protein 1 | 4503801 | 67530 | 70900 | 7.2 | 6.5 |
| Bnf5 | FUSE binding protein 1 GAP.SH3 binding protein 2 | 4503801 3098601 | 67530 50750 | 70600 | 7.2 5.3 | 6.7 |
| Bnf6 | Splicing factor 1 | 1620403 | 68630 | 76000 | 9.3 | 7.5 |
| Bnf7 | HCA56 | 7678701 | 64730 | 75800 | 7.8 | 7.8 |
| Bnf8 | IGF-II mRNA-binding protein 3 | 4191612 | 63690 | 69100 | 9.2 | 8.2 |
| Bnf9 | Hydroxyacyl-CoA dehydrogenase/3-ketoacyl-CoA thiolase/enoyl-CoA hydratase | 4504325 | 82960 | 70600 | 9.2 | 8.9 |
| Bnf10 | Poly(A)-binding protein cytoplasmic 4 | 4504715 | 70760 | 70600 | 9.5 | 9.2 |
| Bnf11 | IGF-11 mRNA-binding protein 1 | 5729882 | 63460 | 65700 | 9.5 | 9.2 |
| Bnf12 | IGF-II mRNA-binding protein 1 | 5729882 | 63460 | 65700 | 9.5 | 9.1 |
| Bnf13 | IGF-II mRNA-binding protein 3 | 4191612 | 63690 | 68900 | 9.2 | 8.5 |
| Bnf14 | NS-associated protein 1 | 5453806 | 62640 | 67900 | 6.9 | 7.6 |
| Bnf15 | Gap SH3 binding protein | 5031703 | 52150 | 70000 | 5.4 | 5.4 |
| Bnf16 | Gap SH3 binding protein | 5031703 | 52150 | 70000 | 5.4 | 5.5 |
| Bnf17 | HnRNP K | 4504453 | 51050 | 65100 | 5.1 | 5.4 |
| Bnf18 | ATP synthase beta chain | 114549 | 56640 | 56600 | 5.3 | 5.0 |
| Bnf19 | ER-60 | 4885359 | 56770 | 54100 | 5.9 | 6.1 |
| Bnf20 | Tat binding protein 1 | 4506211 | 45150 | 46200 | 5.3 | 5.1 |
| Bnf21 | Cargo selection protein | 81347 | 47030 41790 | 40000 | 5.3 | 5.1 |
| | Gamma-actin | 4741428 | | | 5.3 | |
| Bnf22 | Elongation initiation factor 3, subunit 4 | 2460200 | 35.590 | 46.200 | 5.9 | 6.1 |
| Bnf23 | Hn RNP 1 | 131528 | 57200 | 53700 | 9.3 | 8.5 |
| Bnf24 | Hn RNP 1 | 131523 | 57200 | 53900 | 9.3 | 8.7 |
| Bnf25 | Hn RNP 1 | 131528 | 57200 | 53700 | 9.3 | 9.0 |
| Bnf26 | Hn RNP 1 | 131528 | 57200 | 53700 | 9.3 | 9.1 |
| Bnf27 | Hn RNP 1 | 131528 | 57200 | 53000 | 9.3 | 9.4 |
| Bnf28 | DAZ associated protein 1 | 9506537 | 43410 | 48200 | 9.0 | 8.1 |
| | | | | | | |
| Bnf29 | Elongation initiation factor 3, subunit 4 | 2460200 | 35590 | 41200 | 5.9 | 6.2 |
| Bnf30 | HnRNP C1/C2 | 4758544 | 31950 | 35300 | 5.1 | 5.3 |
| Bnf31 | HNRNP A0 | 8134660 | 30840 | 35600 | 9.3 | 9.9 |
| Bnf32 | Lactate dehydrogenase A | 5031857 | 36690 | 35400 | 8.4 | 8.2 |
| Bnf33 | RhoGDI 2 | 1707893 | 22990 | 23100 | 5.1 | 5.1 |
| Bnf34 | ATP synthase D chain | 6831494 | 18360 | 23000 | 5.2 | 5.2 |
| Bnf35 | TF BTF3 | 29507 | 17680 | 19000 | 6.8 | 7.7 |

**Table 8**

| **Table 8 shows proteins of the total cell lysate, the membrane and the mitochondrial fraction. Apoptosis was induced by Fas.** | | | | | | |
|---|---|---|---|---|---|---|
| **Proteins of the total lysate, cis-platin induced** | | | | | | |
| **Spot** | **Protein** | **NCBI** | **Mr theor.** | **Mr found** | **pI theor.** | **pI found** |
| PP1 | Alpha-Fodrin | 4507191 | 284.26 | 84.50 | 5.2 | 5.7 |
| PP2 | Hsp-60 | 306890 | 61.04 | 45.90 | 5.7 | 6.0 |
| NP1 | Chondrosarcoma-associsted protein 2 | 5901878 | 65.57 | 78.90 | 6.3 | 6.5 |
| NP2 | ELAV-like 1 (Hu antigen R) | 4503551 | 36.04 | 35.30 | 9.4 | 9.4 |
| NP3 | HnRNP M | 5174611 | 59.95 | 63.30 | 9.0 | 7.9 |
| NP4 | HnRMP E1 | 1215671 | 37.48 | 41.00 | 6.7 | 6.9 |
| NPS | SKI interacting protein | 6912675 | 61.49 | 64.00 | 9.5 | 9.5 |
| NP6 | Glutathione S-transferase | 31948 | 23.21 | 23.00 | 5.4 | 5.7 |
| NP7 | VDAC3 | 5032221 | 30.64 | 33.40 | 9.2 | 8.8 |
| | | | | | | |
| **Proteins of the membrane, cis-platin induced** | | | | | | |
| **Spot** | **Protein** | **NCBI** | **Mr theor.** | **Mr found** | **pI theor.** | **pI found** |
| NP1 | Mortalin-2 (Heat shock 70kd protein 9B) | 4758570 | 73.78 | 69.60 | 5.97 | 5.40 |
| NP2 | Prohibitin | 4505773 | 29.80 | 26.20 | 5.57 | 5.60 |
| | | | | | | |
| **Proteins of the mitochondrion, cis-platin induced** | | | | | | |
| **Spot** | **Protein** | **NCBI** | **Mr theor.** | **Mr found** | **pI theor.** | **pI found** |
| NP1 | 26S protease regulatory subunit 4 | 345717 | 49.24 | 56.80 | 5.77 | 6.00 |
| NP2 | Proteasome subunit alpha type 1 | 13543551 | 29.58 | 32.50 | 6.15 | 6.20 |

The disclosed subject-matter relates to the following items:
1. A method for characterizing and/or identifying apoptosis-modified proteins comprising the steps:
   (a) providing a first extract and a second extract comprising soluble proteins, wherein said first extract is from a cell without apoptosis induction and said second extract is from a cell after apoptosis induction,
   (b) separating said first and second extracts by two-dimensional gel electrophoresis, wherein first and second proteome patterns each comprising a plurality of protein species are obtained,
   (c) comparing said first and second proteome patterns and
   (d) characterizing and/or identifying apoptosis-modified protein species.
2. The method of item 1, wherein after apoptosis induction substantially no synthesis of new proteins has been allowed.
3. The method of item 2, wherein the protein biosynthesis has been substantially blocked by an inhibitor.
4. The method of items 2 or 3, wherein apoptosis induction has been carried 4 out for a period of time which is too short to allow a substantial synthesis of new proteins.
5. The method of any one of items 1-4, wherein said two-dimensional gel electrophoresis comprises (i) separation in a first dimension according to the isoelectric point and (ii) separation in a second dimension according to size.
6. The method of any one of items 1-5, wherein the apoptosis-modified protein species are selected from protein species which (i) are located at different positions on the two-dimensional gels from the first and second extracts and/or (ii) have a different intensity on the two-dimensional gels from the first and second extracts.
7. The method of any one of items 1-6, wherein the protein species are characterized by peptide fingerprinting.
8. The method of item 7, wherein the peptides are characterized by mass spectrometry and/or at least partial sequencing.
9. The method of any one of item 1-8, wherein said cell is a mammalian cell.
10. The method of item 9, wherein said cell is a human cell.
11. The method of item 9 or 10, wherein said cell is a T-cell.
12. The method of item 11, wherein said T-cell is the T-cell line Jurkat E6 (ATCC TIB 152).
13. The method of any one of items 1-12, wherein the apoptosis is induced by an anti-Fas antibody or by treatment with cis-platin.
14. The method of any one of items 1-13, wherein the apoptosis-modified protein species are selected from heterogeneous nuclear ribonucleoproteins, splicing factors, translation factors, structural proteins, signal transduction proteins, chromatin associated proteins, transcription factors, proteasome subunits, mitochondrial proteins, nucleophosmin, SYT interacting protein SIP, PA1-G, CRHSP-24, HCD2, GMP synthase, FUSE binding protein 1, HDGF, PFC6D, KPF1, KNFE3 having the partial sequence TPGT (F/Mox)E, alpha NAC, ARDH, cargo selection protein, DAZ associated protein 1, DEAD box protein retinoblastoma, dihydrofolate reductase, hydroxyacyl-CoA dehydrogenase/3-ketoacyl-CoA thiolase/enoyl-CoA hydratase, ER-60, HCA56, Hsp-105, IGF-II mRNA-binding protein 1, IGF-II mRNA-binding protein 3, lactate dehydrogenase A, NS-associated protein, RAD 21, RAD 23 homolog B, T-complex protein 1 beta subunit, thioredoxin like protein, an unnamed protein (NCBI 7020309), and c-Abl or a partial sequence derived therefrom by substitution and/or deletion of one or more amino acids.
15. The method of any one of items 1-14 further comprising
   (e) determining if the apoptosis-modified proteins are present in subjects suffering from apoptosis-associated diseases.
16. Apoptosis-associated and/or -modified protein selected from heterogeneous nuclear ribonucleoproteins, splicing factors, translation factors, structural proteins, signal transduction proteins, chromatin associated proteins, transcription factors, proteasome subunits, mitochondrial proteins, nucleophosmin, SYT interacting protein SIP, PA1-G, CRHSP-24, HCD2, GMP synthase, FUSE binding protein 1, HDGF, PFC6D, KPF1, KNFE3 having the partial sequence TPGT (F/Mox)E, alpha NAC, ARDH, cargo selection protein, DAZ associated protein 1, DEAD box protein retinoblastoma, dihydrofolate reductase, hydroxyacyl-CoA dehydrogenase/3-ketoacyl-CoA thiolase/enoyl-CoA hydratase, ER-60, HCA56, Hsp-105, IGF-II mRNA-binding protein 1, IGF-II mRNA-binding protein 3, lactate dehydrogenase A, NS-associated protein, RAD 21, RAD 23 homolog B, T-complex protein 1 beta subunit, thioredoxin like protein, an unnamed protein (NCBI 7020309) and c-Abl or a partial sequence derived therefrom by substitution and/or deletion of one or more amino acids.
17. Apoptosis-associated and/or -modified protein selected from the proteins as shown in Table 1, 2, 3, 4, 5, 6, 7 or 8 or proteolytic fragments thereof.
18. Use of a protein of items 16 or 17 as target for the diagnosis, prevention or treatment of apoptosis-associated diseases or in a method for identifying apoptosis modulators.

## Claims

1. A screening method for identifying an apoptosis modulator,
**characterized in that**
the method comprises determination of transcriptional, translational, posttranslational variation and/or translocation from one compartment to another of prohibitin in a cell during apoptosis,
wherein the apoptosis modulator is capable of modifying prohibitin during apoptosis.

2. The method of claim 1, wherein the modulator is an activator of apoptosis.

3. The method of claim 1, wherein the modulator is an inhibitor of apoptosis.

4. The method of any of the preceding claims, wherein apoptosis is induced by an anti-Fas antibody or by cisplatin.

5. The method of any of the preceding claims, wherein prohibitin changes in size or/and charge after apoptosis induction.

6. The method of any of the claims 1 to 5, wherein prohibitin is changed by transcriptional, translational and/or posttranslational variation after apoptosis induction.

7. The method of any of the claims 1 to 5, wherein prohibitin is translocated after apoptosis induction.

8. The method of claim 7, wherein prohibitin is translocated from the membrane compartment after apoptosis induction.

9. The method of any of the preceding claims, wherein the cell is a mammalian cell.

10. The method of any of the preceding claims, wherein the cell is a human cell.

11. The method of any of the preceding claims which is performed in the high throughput format.

12. The method of any of the claims 1 to 11, wherein protein biosynthesis is substantially blocked.

13. The method of any of the claims 1 to 11, wherein apoptosis induction is carried out for a period of time which is too short to allow a substantial synthesis of new proteins.

14. Use of prohibitin in a method for identifying apoptosis modulators,
wherein the apoptosis modulator is capable of modifying prohibitin during apoptosis.

15. Use of claim 14, wherein the method is the method of any of the claims 1 to 13.

## Patentansprüche

1. Screeningverfahren zur Identifizierung eines Apoptosemodulators,
**dadurch gekennzeichnet, dass**
das Verfahren die Bestimmung der transkriptionalen, translationalen, posttranslationalen Variation und/oder der Translokation aus einem Kompartiment in ein anderes von Prohibitin in einer Zelle während Apoptose umfasst,
wobei der Apoptosemodulator fähig ist, Prohibitin während Apoptose zu modulieren.

2. Verfahren gemäß Anspruch 1, wobei der Modulator ein Aktivator von Apoptose ist.

3. Verfahren gemäß Anspruch 1, wobei der Modulator ein Inhibitor von Apoptose ist.

4. Verfahren gemäß einem der vorhergehenden Ansprüche, wobei Apoptose durch einen anti-Fas-Antikörper oder durch Cisplatin induziert wird.

5. Verfahren gemäß einem der vorhergehenden Ansprüche, wobei Prohibitin nach Apoptoseinduktion in der Größe oder/und Ladung verändert wird.

6. Verfahren gemäß einem der Ansprüche 1 bis 5, wobei Prohibitin nach Apoptoseinduktion durch transkriptionale, translationale und/oder posttranslationale Variation verändert wird.

7. Verfahren gemäß einem der Ansprüche 1 bis 5, wobei Prohibitin nach Apoptoseinduktion transloziert wird.

8. Verfahren gemäß Anspruch 7, wobei Prohibitin nach Apoptoseinduktion aus dem Membrankompartiment transloziert wird.

9. Verfahren gemäß einem der vorhergehenden Ansprüche, wobei die Zelle eine Säugerzelle ist.

10. Verfahren gemäß einem der vorhergehenden Ansprüche, wobei die Zelle eine menschliche Zelle ist.

11. Verfahren gemäß einem der vorhergehenden Ansprüche, welches im Hochdurchsatzformat durchgeführt wird.

12. Verfahren gemäß einem der Ansprüche 1 bis 11, wobei die Proteinbiosynthese im wesentlichen blockiert wird.

13. Verfahren gemäß einem der Ansprüche 1 bis 11, wobei die Apoptoseinduktion für eine Zeitdauer durchgeführt wird, welche zu kurz ist, um eine wesentliche Synthese neuer Proteine zu erlauben.

14. Verwendung von Prohibitin in einem Verfahren zur Identifizierung von Apoptosemodulatoren, wobei der Apoptosemodulator fähig ist, Prohibitin während Apoptose zu modulieren.

15. Verwendung gemäß Anspruch 14, wobei das Verfahren ein Verfahren gemäß einem der Ansprüche 1 bis 13 ist.

## Revendications

1. Procédé de criblage pour l'identification d'un modulateur de l'apoptose,
**caractérisé en ce que**
le procédé comprend la détermination d'une variation et/ou d'une translocation transcriptionnelle, traductionnelle, post-traductionnelle d'un compartiment à un autre de la prohibitine dans une cellule pendant l'apoptose,
le modulateur de l'apoptose étant capable de modifier la prohibitine pendant l'apoptose.

2. Procédé selon la revendication 1, dans lequel le modulateur est un activateur de l'apoptose.

3. Procédé selon la revendication 1, dans lequel le modulateur est un inhibiteur de l'apoptose.

4. Procédé selon l'une quelconque des revendications précédentes, dans lequel l'apoptose est induite par un anticorps anti-Fas ou par le cisplatine.

5. Procédé selon l'une quelconque des revendications précédentes, dans lequel la prohibitine change de taille et/ou de charge après induction de l'apoptose.

6. Procédé selon l'une quelconque des revendications 1 à 5, dans lequel la prohibitine est modifiée par une variation transcriptionnelle, traductionnelle et/ou post-traductionnelle après induction de l'apoptose.

7. Procédé selon l'une quelconque des revendications 1 à 5, dans lequel la prohibitine est soumise à une translocation après induction de l'apoptose.

8. Procédé selon la revendication 7, dans laquelle la prohibitine est soumise à une translocation du compartiment de membrane après induction de l'apoptose.

9. Procédé selon l'une quelconque des revendications précédentes, dans lequel la cellule est une cellule de mammifère.

10. Procédé selon l'une quelconque des revendications précédentes, dans lequel la cellule est une cellule humaine.

11. Procédé selon l'une quelconque des revendications précédentes, qui est réalisé à un format de débit élevé.

12. Procédé selon l'une quelconque des revendications 1 à 11, dans lequel la biosynthèse de la protéine est sensiblement bloquée.

13. Procédé selon l'une quelconque des revendications 1 à 11, dans lequel l'induction de l'apoptose est réalisée pendant une durée qui est trop courte pour permettre une synthèse substantielle de nouvelles protéines.

14. Utilisation de la prohibitine dans un procédé servant à identifier des modulateurs de l'apoptose, le modulateur de l'apoptose étant capable de modifier la prohibitine pendant l'apoptose.

15. Utilisation selon la revendication 14, dans laquelle le procédé est le procédé selon l'une quelconque des revendications 1 à 13.
